# EUROPEAN PATENT APPLICATION

(11) **EP 4 431 503 A1**
(43) Date of publication of application: **18.09.2024**
(21) Application number: 22892003.9
(22) Date of filing: 09.11.2022
(51) Int. Cl.: C07D 401/12, C07F 5/00, A61K 51/04, A61P 35/00

(54) **LIGAND COMPOUND TARGETING PSMA ANTIGEN, AND CHELATE AND USE THEREOF IN DIAGNOSIS AND TREATMENT OF PROSTATE CANCER**

(30) Priority: 10.11.2021 CN 202111329108
(71) Applicant: Suzhou Ruihe Medicine Technology Co., Ltd, Suzhou, Jiangsu 215000 (CN)
(72) Inventor: ZHONG, Zhiyuan, Suzhou, Jiangsu 215000 (CN); YUAN, Jiandong, Suzhou, Jiangsu 215000 (CN); YANG, Jiangtao, Suzhou, Jiangsu 215000 (CN); HUANGFU, Zhenyuan, Suzhou, Jiangsu 215000 (CN); SUN, Juan, Suzhou, Jiangsu 215000 (CN); XU, Bin, Suzhou, Jiangsu 215000 (CN); TAO, Lei, Suzhou, Jiangsu 215000 (CN)
(74) Representative: Perani & Partners S.p.A.
(86) International application number: PCT/CN2022/130833
(87) International publication number: WO 2023/083209

(57) **Abstract**

Disclosed are a ligand compound targeting a PSMA antigen, and a chelate and the use thereof in the diagnosis and treatment of prostate cancer. The ligand compound is a compound of formula (I) or a salt, ester or solvate thereof. The present invention relates to the technical field of radiopharmaceuticals. A chelate containing the ligand compound of the present invention and a radioactive metal can be used as a PSMA targeted radiopharmaceutical with a longer blood half-life, a better antigen affinity and tumor inhibition effect, a lower toxicity and a higher safety, and is used for diagnosing and treating PSMA antigen positive prostate cancer in nuclear medicine.

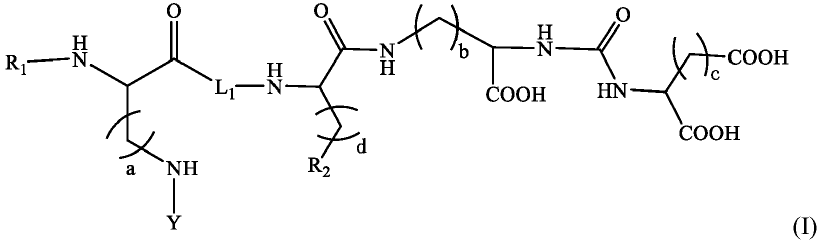

## Description

### Cross-Reference to Related Applications

The present invention claims the right of priority for patent application no. 202111329108.1 titled "LIGAND COMPOUND TARGETING PSMA ANTIGEN, AND PREPARATION METHOD THEREFOR AND USE THEREOF IN DIAGNOSIS AND TREATMENT OF PROSTATE CANCER" and filed with the China Patent Office on November 10, 2021, the entire content of which is incorporated herein by reference in its entirety.

### Technical Field

The present invention belongs to the field of pharmaceutical chemistry, and particularly relates to a ligand compound targeting a PSMA antigen, a chelate thereof, and the use thereof as a reagent for diagnosing prostate cancer in nuclear medicine and the use thereof in the treatment of varying degrees of prostate cancer.

### Background Art

There were 1,414,259 new cases of patients with prostate cancer worldwide in 2020, accounting for 7.3% of all new cancer patients, the third highest, and the prostate cancer is a cancer of the highest incidence in men. There were 375,304 new deaths in patients with prostate cancer worldwide in 2020, accounting for 3.8% of all cancer deaths. This benefits from a large number of current treatments, including castration surgery, external beam radiotherapy, brachytherapy, endocrine therapy, chemotherapy, *etc.,* for prostate cancer, but still a significant fraction of patients develops metastatic castration-resistant prostate cancer (mCRPC) after a series of treatments. For these patients, radiopharmaceuticals targeting prostate-specific membrane antigen (PSMA) have a good therapeutic effect. In particular, ¹⁷⁷Lu-PSMA-617 has recently been rated as a breakthrough therapy by the FDA.

Although ¹⁷⁷Lu-PSMA-617 has a good treatment effect on metastatic castration-resistant prostate cancer, its blood half-life is short and its retention time at the tumor site is short. For this reason, many researchers have greatly increased the half-life of drugs by adding side chains that bind to albumin in the drug structures, thus increasing the radiation exposure in tumors. However, this method also greatly increases the radiation exposure in normal tissues at the same time, and there is a risk of toxic and side effects. Therefore, it is necessary to further develop compounds that can greatly increase the radiation exposure in tumors while still have the low radiation exposure in normal tissues, so as to improve the diagnosis or treatment effect without increasing toxic and side effects. There is a need in the art for a radiopharmaceutical targeting prostate-specific membrane antigen (PSMA) with longer blood half-lives, better efficacy, and lower toxicity.

### Summary of the Invention

### Problems to be solved by the invention

To solve the technical problems existing in the prior art, the present invention discloses a novel ligand compound targeting PSMA and a chelate formed by binding the ligand compound to a radioactive metal, the ligand compound or the chelate can be used as a reagent for treating or diagnosing prostate cancer in nuclear medicine, for use in the treatment or diagnosis of varying degrees of prostate cancer, especially PSMA positive prostate cancer.

### Solutions to solve problems

The present invention is implemented by means of the following technical solutions:

The present invention discloses a ligand compound targeting a PSMA antigen, or a pharmaceutically acceptable salt or ester thereof or a solvate thereof, wherein the ligand compound targeting the PSMA antigen has the following chemical structural formula: 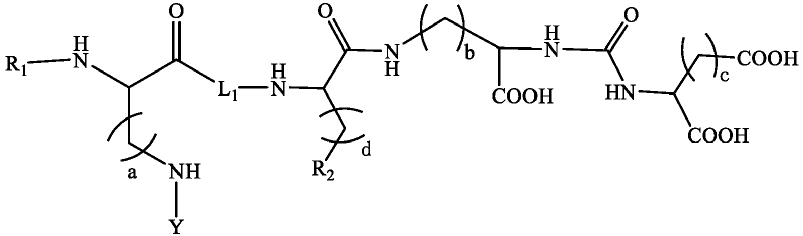 wherein a and b are each independently any integer of 1 to 8, and c and d are each independently any integer of 1 to 4; preferably, a and b are each independently any integer of 2 to 6, and c and d are each independently any integer of 1 to 3; more preferably, a and b are 4, c is 2, and d is 1;

L₁ has any one of the following chemical structural formulas: 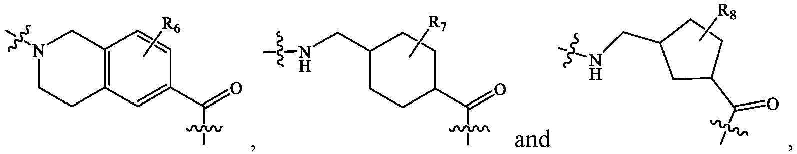 and preferably, L₁ is linked to other fragments in the structural formula of the ligand compound via amide bonds;
wherein R₆, R₇, and R₈ are each independently any one of hydrogen, alkyl, alkoxy, and halogen;
R₁ has any one of the following chemical structural formulas: 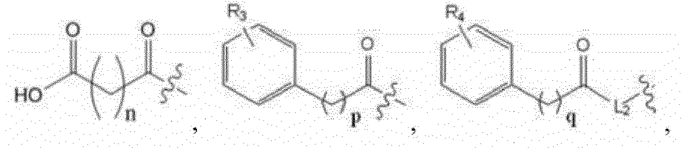 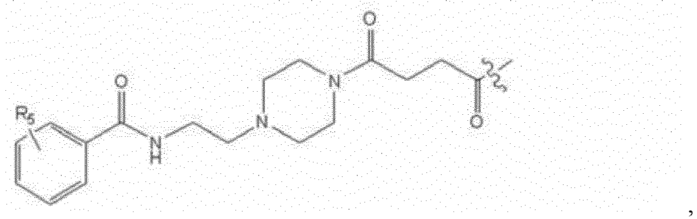
wherein n is any integer of 2 to 20; R₃, R₄, and R₅ are each independently any one of hydrogen, alkyl, alkoxy, halogen, cycloalkyl, heterocyclyl, and aryl; p is any integer of 0 to 8; q is any integer of 1 to 10; L₂ is an amino acid linker structure;
R₂ is aryl or heteroaryl, preferably aryl; Y is a radioactive metal chelator group.

The present invention discloses a ligand compound targeting a PSMA antigen, or a pharmaceutically acceptable salt or ester thereof or a solvate thereof, wherein the ligand compound targeting the PSMA antigen has the following chemical structural formula: 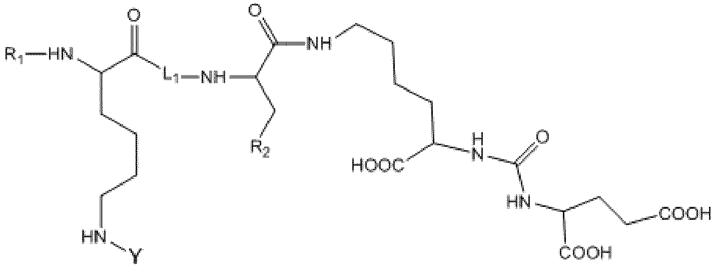 wherein L₁ has any one of the following chemical structural formulas: 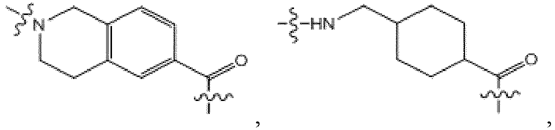 and preferably, L₁ is linked to other fragments in the structural formula of the ligand compound via amide bonds;

R₁ has any one of the following chemical structural formulas: 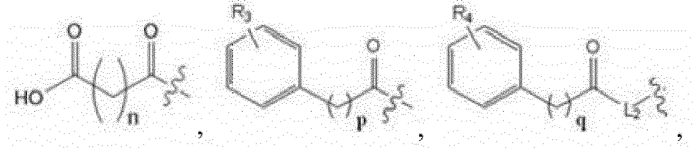 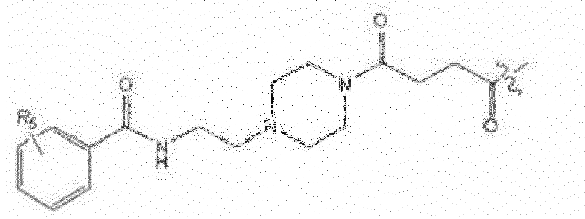
wherein n is any integer of 2 to 20; R₃, R₄, and R₅ are each independently selected from one of hydrogen, alkyl, alkoxy, halogen, cycloalkyl, heterocyclyl, and aryl; p is any integer of 0 to 8; q is any integer of 1 to 10;
L₂ is an amino acid linker structure; R₂ is aryl or heteroaryl, preferably aryl; Y is a radioactive metal chelator group.

In the above-mentioned technical solution, Y is a radioactive metal chelator group, and can bind to a radioactive metal; preferably, the chelator is selected from one of: DOTA (1,4,7,10-tetraazacyclododecane-1,4,7,10-tetraacetic acid) or a derivative thereof, TETA (1,4,8,11-tetraazacyclotetradecane-1,4,8,11-tetraacetic acid) or a derivative thereof, SarAR (1-N-(4-aminobenzyl)-3,6,10,13,16,19-hexaazabicyclo[6.6.6]-eicosane-1,8-diamine) or a derivative thereof, NOTA (1,4,7-triazacyclononane-1,4,7-triacetic acid) or a derivative thereof, TRAP (1,4,7-triazacyclononane-1,4,7-trimethyl(2-carboxyethyl)phosphinic acid) or a derivative thereof, HBED (N,N'-bis(2-hydroxybenzyl)-ethylenediamine-N,N'-diacetic acid) or a derivative thereof, 2,3-HOPO (3-hydroxypyridin-2-one) or a derivative thereof, PCTA (3,6,9,15-tetraazabicyclo[9.3.1]-pentadeca-1(15),11,13-triene-3,6,9-triacetic acid) or a derivative thereof, DFO (deferoxamine) or a derivative thereof, DTPA (diethylenetriamine pentaacetic acid) or a derivative thereof, OCTAPA (N,N'-bis(6-carboxy-2-pyridylmethyl)-ethylenediamine-N,N'-diacetic acid) or a derivative thereof, H2-MACROPA (N,N'-bis[(6-carboxy-2-pyridyl)methyl]-4,13-diaza-18-crown-6) or a derivative thereof.

The present invention discloses a DOTA-based ligand compound targeting a PSMA antigen, or a pharmaceutically acceptable salt or ester thereof or a solvate thereof, wherein the DOTA-based ligand compound targeting the PSMA antigen has the following chemical structural formula: 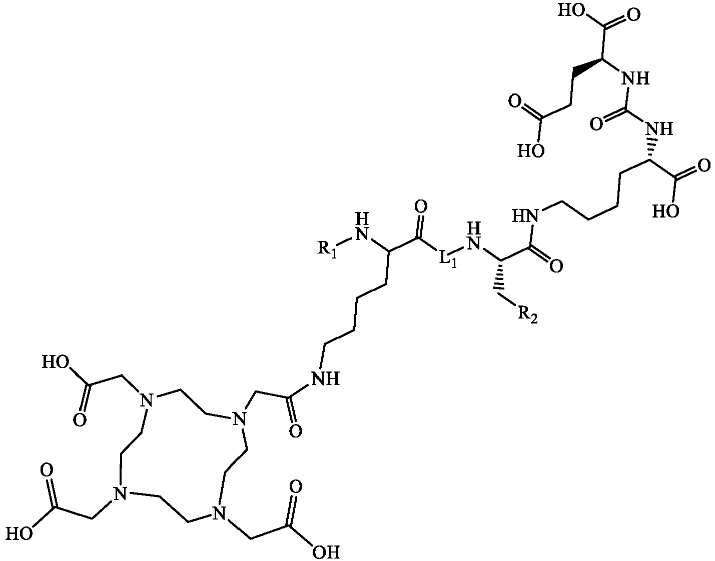
wherein L₁ has any one of the following chemical structural formulas: 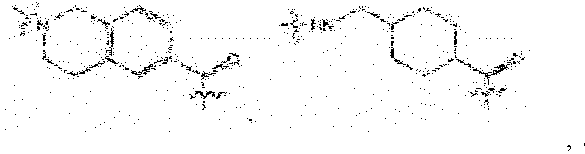 and preferably, L₁ is linked to other fragments in the structural formula of the ligand compound via amide bonds;
R₁ has any one of the following chemical structural formulas: 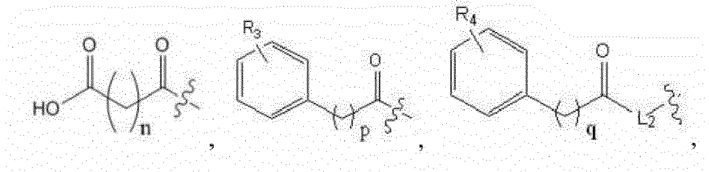 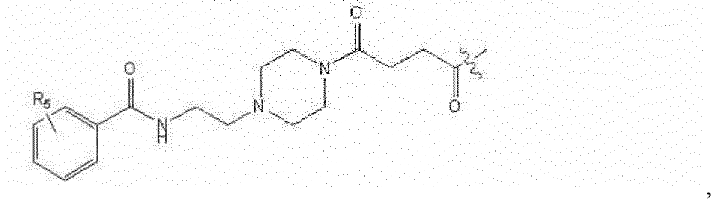
wherein n is any integer of 2 to 20; R₃, R₄, and R₅ are each independently any one of hydrogen, alkyl, alkoxy, halogen, cycloalkyl, heterocyclyl, and aryl; p is any integer of 0 to 8; q is any integer of 1 to 10; L₂ is an amino acid linker structure;
R₂ is aryl or heteroaryl, preferably aryl, more preferably naphthyl.

Preferably, the DOTA-based ligand compound targeting PSMA antigen has the following chemical structural formula: 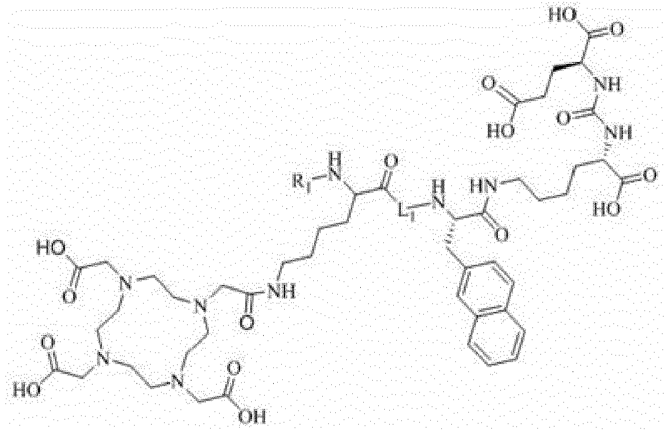 wherein L₁, R₁ are as defined in the chemical structural formula of the DOTA-based ligand compound targeting PSMA antigen.

In the above-mentioned technical solution, the aryl is phenyl, naphthyl or anthryl.

In the above-mentioned technical solution, preferably, the n is any integer of 4 to 18, the alkoxy is alkoxy containing 1 to 10 carbon atoms, the alkyl is alkyl containing 1 to 10 carbon atoms, the q is any integer of 2 to 8, and the p is any integer of 0 to 6;
more preferably, the n is any integer of 6 to 16, the alkoxy is alkoxy containing 1 to 6 carbon atoms, the alkyl is alkyl containing 1 to 6 carbon atoms, the q is any integer of 2 to 5, and the p is any integer of 0 to 4;
further preferably, the n is any integer of 8 to 14, the alkoxy is alkoxy containing 1 to 3 carbon atoms, the alkyl is alkyl containing 1 to 3 carbon atoms, the q is 2 or 3, and the p is 0 or 1.

In the present invention, the amino acid linker structure refers to the remaining structure of an amino acid in which a hydroxyl group is removed from the terminal carboxyl group and one hydrogen is removed from an amino group linked to the methylene group adjacent to the terminal carboxyl group; the amino group on the amino acid linker and a phenylalkanoic acid group form an amide bond, constituting R₁ structure; the amino acid is arginine, serine, histidine, lysine, glycine or glutamine.

Preferably, the R₁ has the following chemical structural formula: 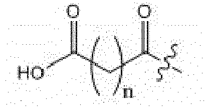 wherein the n is any integer of 4 to 18, preferably any integer of 6 to 16, more preferably any integer of 8 to 14.

Preferably, the R₁ has the following chemical structural formula: 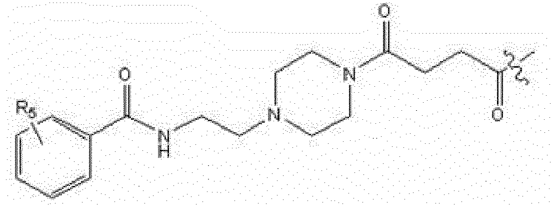 wherein R₅ is hydrogen, alkoxy containing 1 to 6 carbon atoms or alkyl containing 1 to 6 carbon atoms; more preferably, the alkoxy is alkoxy containing 1 to 3 carbon atoms, and the alkyl is alkyl containing 1 to 3 carbon atoms.

Preferably, the R₁ has the following chemical structural formula: 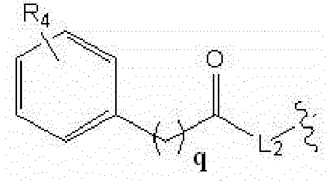
wherein R₄ is hydrogen, alkoxy containing 1 to 6 carbon atoms or alkyl containing 1 to 6 carbon atoms, q is any integer of 2 to 5, and L₂ is an amino acid linker structure;
more preferably, the alkoxy is alkoxy containing 1 to 3 carbon atoms, the alkyl is alkyl containing 1 to 3 carbon atoms, q is 2 or 3, and the amino acid in the amino acid linker is arginine, serine, histidine, lysine, glycine or glutamine.

Preferably, the R₁ has the following chemical structural formula: 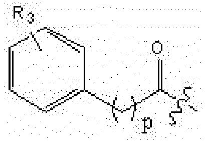
wherein R₃ is hydrogen, alkoxy or alkyl; p is any integer of 0 to 7;
preferably, the alkoxy is alkoxy containing 1 to 10 carbon atoms, and the alkyl is alkyl containing 1 to 10 carbon atoms; p is any integer of 0 to 4;
further preferably, the alkoxy is alkoxy containing 1 to 6 carbon atoms, and the alkyl is alkyl containing 1 to 6 carbon atoms; p is any integer of 0 to 2;
most preferably, the alkoxy is alkoxy containing 1 to 3 carbon atoms, and the alkyl is alkyl containing 1 to 3 carbon atoms; the p is 0 or 1.

As a preferred example, the ligand compound targeting PSMA antigen or the DOTA-based ligand compound targeting PSMA antigen is one of PSMA-A, PSMA-B, PSMA-C, PSMA-D, PSMA-E, PSMA-F, PSMA-G, PSMA-H, PSMA-1, PSMA-J, PSMA-K, and PSMA-L.

As a particularly preferred example, the present invention discloses a ligand compound having the following structural formula or a pharmaceutically acceptable salt or ester thereof or a solvate thereof:

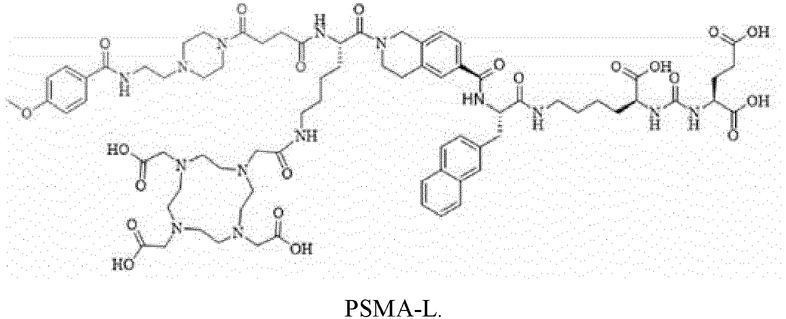

The present invention discloses a chelate, which comprises a ligand compound or a pharmaceutically acceptable salt or ester thereof or a solvate thereof, and a radioactive metal. The ligand compound is the above-mentioned ligand compound targeting PSMA antigen or the above-mentioned DOTA-based ligand compound targeting PSMA antigen, or the ligand compound as the particularly preferred example. The radioactive metal binds to the above-mentioned ligand compound through the radioactive metal chelator group Y, wherein the radioactive metal is ⁶⁴Cu, ⁶⁷Cu, ⁹⁰Y, ¹¹¹In, ⁶⁸Ga, ^{117m}Sn, ¹⁵³Sm, ¹⁴⁹Tb, ¹⁶¹Tb, ¹⁷⁷Lu, ²²⁵Ac, ²¹³Bi, ²²⁴Ra, ²¹²Bi, ²¹²Pb, ²²⁵Ac, ²²⁷Th, ²²³Ra, ⁴⁷Sc, ¹⁸⁶Re, ¹⁸⁸Re, ⁶⁸Ga, ⁶⁴Cu, ¹¹¹In, ⁸⁹Zr, ⁴⁴Sc, ^{99m}Tc, ⁸⁶Y, ¹⁵²Tb or ¹⁵⁵Tb.

In some embodiments, when the radioactive metal is a diagnostic radioactive metal (including but not limited to ⁶⁸Ga, ⁶⁴Cu, ¹¹¹In, ⁸⁹Zr, ⁴⁴Sc, ^{99m}Tc, ⁸⁶Y, ¹⁵²Tb or ¹⁵⁵Tb), the chelate can be used for the diagnosis of prostate cancer, for use in the imaging diagnosis of patients with PSMA positive prostate cancer; In some embodiments, when the radioactive metal is a therapeutic radioactive metal (including but not limited to ⁶⁴Cu, ⁶⁷Cu, ⁹⁰Y, ¹¹¹In, ^{117m}Sn, ¹⁵³Sm, ¹⁴⁹Tb, ¹⁶¹Tb, ¹⁷⁷Lu, ²²⁵Ac, ²¹³Bi, ²²⁴Ra, ²¹²Bi, ²¹²Pb, ²²⁵Ac, ²²⁷Th, ²²³Ra, ⁴⁷Sc, ¹⁸⁶Re or ¹⁸⁸Re), these compounds can be used for the treatment of prostate cancer, for use in the treatment of patients with PSMA positive prostate cancer.

As a preferred example, the radioactive metal is ¹⁷⁷Lu.

As a preferred example, the chelate comprises ¹⁷⁷Lu and a ligand compound having the following structure or a pharmaceutically acceptable salt or ester thereof or a solvate thereof:

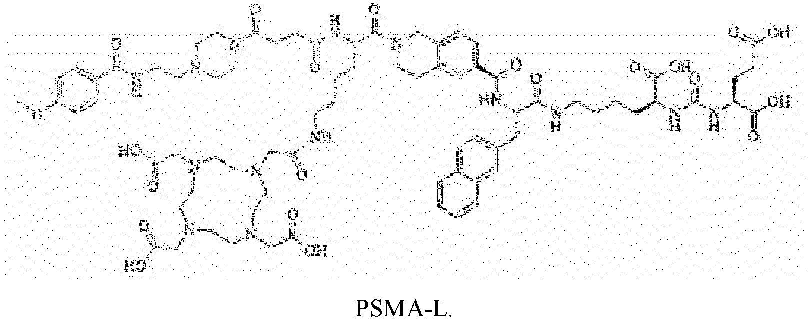

A composition comprises the above-mentioned chelate, and can further comprise a pharmaceutically acceptable excipient.

A cancer diagnosis or treatment reagent comprises the above-mentioned chelate as an active ingredient, and can further comprise a pharmaceutically acceptable excipient.

The present invention discloses the use of the above-mentioned composition, the above-mentioned chelate, and the above-mentioned ligand compound or the pharmaceutically acceptable salt or ester thereof or the solvate thereof in the preparation of a cancer diagnosis or treatment reagent. The ligand compound is the above-mentioned ligand compound targeting PSMA antigen or the above-mentioned DOTA-based ligand compound targeting PSMA antigen, or the ligand compound as the particularly preferred example. Preferably, the diagnosis is an imaging diagnosis; the treatment is radiotherapy; the cancer is a solid tumor or a hematological tumor; further, the cancer is prostate cancer, more preferably prostate-specific membrane antigen positive prostate cancer.

The present invention discloses a method for imaging a patient with prostate cancer, comprising the following steps: administering to the patient the above-mentioned chelate, or the above-mentioned composition, or the above-mentioned cancer diagnosis or treatment reagent, or a composition containing the above-mentioned cancer diagnosis reagent or the above-mentioned cancer treatment reagent and a pharmaceutically acceptable excipient (preferably in a diagnostically effective amount), and then performing tissue imaging; preferably, the prostate cancer is prostate-specific membrane antigen positive prostate cancer; the imaging is PET imaging or SPECT imaging.

The present invention discloses a method for treating a patient with prostate cancer, comprising the following steps: administering to the patient the above-mentioned chelate, or the above-mentioned composition, or the above-mentioned cancer diagnosis or treatment reagent, or a composition containing the above-mentioned cancer diagnosis reagent and a pharmaceutically acceptable excipient (preferably in a diagnostically effective amount); preferably, the prostate cancer is prostate-specific membrane antigen positive prostate cancer; the treatment is radiotherapy.

The present invention discloses the above-mentioned cancer diagnosis or treatment reagent, the above-mentioned chelate, and the above-mentioned ligand compound or the pharmaceutically acceptable salt or ester thereof or the solvate thereof, which are used for diagnosing and treating a cancer; preferably, the diagnosis is imaging (such as PET imaging or SPECT imaging) diagnosis, the treatment is radiotherapy, and the cancer is a solid tumor or a hematological tumor; further, the cancer is prostate cancer, more preferably prostate-specific membrane antigen positive prostate cancer.

### Effects of the Invention

After chelation with a metal, the ligand compound targeting PSMA antigen disclosed in the present invention has a lower toxicity to normal tissues, higher exposure dose at the tumor site, longer half-life in blood, better antigen affinity, better inhibitory effect on tumors, good stability of radiochemical purity and good safety *in vivo,* can be used for diagnosing (such as performing PET or SPECT imaging of) patients with prostate-specific membrane antigen (PSMA) positive prostate cancer, and can further be used for treating patients with prostate-specific membrane antigen positive prostate cancer. The radioactive metal is coordinated with the ligand compound targeting PSMA antigen disclosed in the present invention very stably, and does not fall off *in vitro* and *in vivo,* which is very beneficial for the production, transportation and preservation of drugs, and diagnosis and treatment in the later stage.

### Brief Description of the Drawings

FIG. 1 shows SPECT/CT scanning patterns of ¹⁷⁷Lu-PSMA-617 in LNCaP tumor bearing mice and quantification of radioactive distribution in the tumor and various tissues.
FIG. 2 shows SPECT/CT scanning patterns of ¹⁷⁷Lu-PSMA-A in LNCaP tumor bearing mice and quantification of radioactive distribution in the tumor and various tissues.
FIG. 3 shows SPECT/CT scanning patterns of ¹⁷⁷Lu-PSMA-B in LNCaP tumor bearing mice and quantification of radioactive distribution in the tumor and various tissues.
FIG. 4 shows SPECT/CT scanning patterns of ¹⁷⁷Lu-PSMA-C in LNCaP tumor bearing mice and quantification of radioactive distribution in the tumor and various tissues.
FIG. 5 shows SPECT/CT scanning patterns of ¹⁷⁷Lu-PSMA-E in LNCaP tumor bearing mice and quantification of radioactive distribution in the tumor and various tissues.
FIG. 6 shows SPECT/CT scanning patterns of ¹⁷⁷Lu-PSMA-F in LNCaP tumor bearing mice and quantification of radioactive distribution in the tumor and various tissues.
FIG. 7 shows SPECT/CT scanning patterns of ¹⁷⁷Lu-PSMA-G in LNCaP tumor bearing mice and quantification of radioactive distribution in the tumor and various tissues.
FIG. 8 shows SPECT/CT scanning patterns of ¹⁷⁷Lu-PSMA-H in LNCaP tumor bearing mice and quantification of radioactive distribution in the tumor and various tissues.
FIG. 9 shows SPECT/CT scanning patterns of ¹⁷⁷Lu-PSMA-I in LNCaP tumor bearing mice and quantification of radioactive distribution in the tumor and various tissues.
FIG. 10 shows SPECT/CT scanning patterns of ¹⁷⁷Lu-PSMA-K in LNCaP tumor bearing mice and quantification of radioactive distribution in the tumor and various tissues.
FIG. 11 shows SPECT/CT scanning patterns of ¹⁷⁷Lu-PSMA-L in LNCaP tumor bearing mice and quantification of radioactive distribution in the tumor and various tissues.
FIG. 12 shows the binding of ¹⁷⁷Lu-PSMA-617 and the binding of ¹⁷⁷Lu-PSMA-L to LNCaP cells.
FIG. 13 the pharmacokinetic profiles of ¹⁷⁷Lu-PSMA-617 and ¹⁷⁷Lu-PSMA-L in mice and rats.
FIG. 14 shows the stability of radiochemical purity of ¹⁷⁷Lu-PSMA-L in PBS buffer and the stability of radiochemical purity of ¹⁷⁷Lu-PSMA-L in 10% mouse serum.
FIG. 15 shows the effect of ¹⁷⁷Lu-PSMA-L on leukocytes, hemoglobin and platelets in mouse blood.
FIG. 16 shows the effect of ¹⁷⁷Lu-PSMA-L on the body weight of mice at different administration doses.
FIG. 17 shows the inhibitory effects of ¹⁷⁷Lu-PSMA-617 and ¹⁷⁷Lu-PSMA-L on the tumor in LNCaP tumor bearing mice and the change in the body weight of animals during treatment.
FIG. 18 shows the radioactivity activity at the tumor site and the quantitative value of the radioactivity activity at the tumor site during the inhibition on the tumor in LNCaP tumor bearing mice by ¹⁷⁷Lu-PSMA-617 and ¹⁷⁷Lu-PSMA-L.
FIG. 19 shows a liquid chromatogram of compound PSMA-L.
FIG. 20 shows a mass spectrum of compound PSMA-L.
FIG. 21 shows a nuclear magnetic resonance pattern of compound PSMA-L, ¹H NMR (600 MHz, dmso).
FIG. 22 shows a nuclear magnetic resonance pattern of compound PSMA-H, ¹H NMR (600 MHz, dmso).

### Detailed Description of Embodiments

The present invention discloses a compound of formula (I), or a salt, ester or solvate of the compound, or a pharmaceutically acceptable salt thereof or a solvate thereof; the compound of formula (I) is as follows:

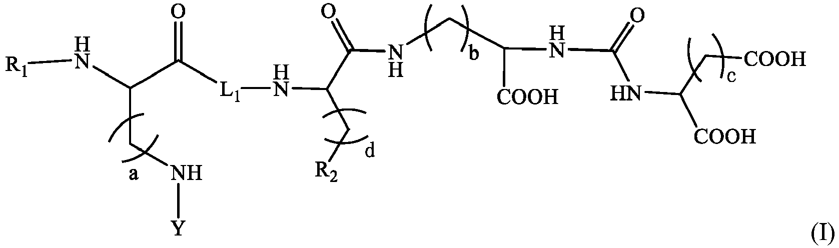

The substituents are as defined above.

PSMA-617 has the following chemical structural formula:

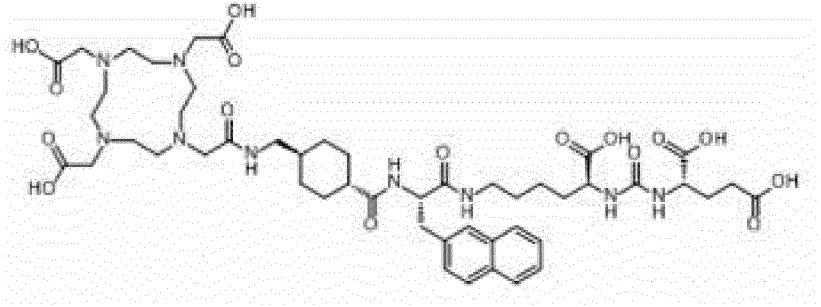

Some of the novel compounds targeting PSMA disclosed in the present invention are exemplified below:
PSMA-A: 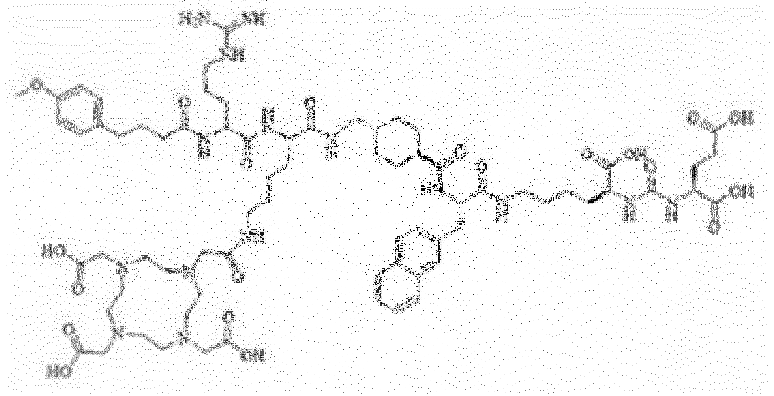
PSMA-B: 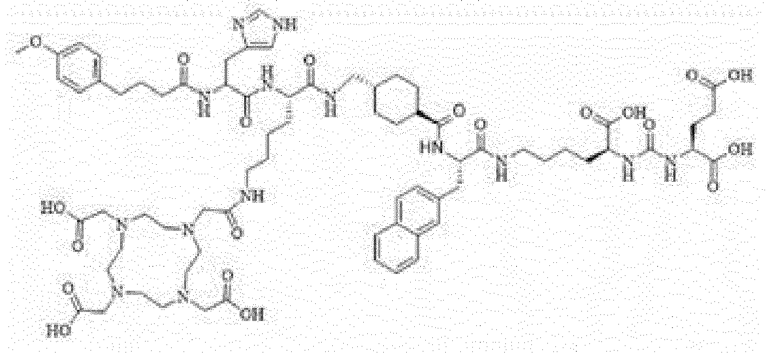
PSMA-C: 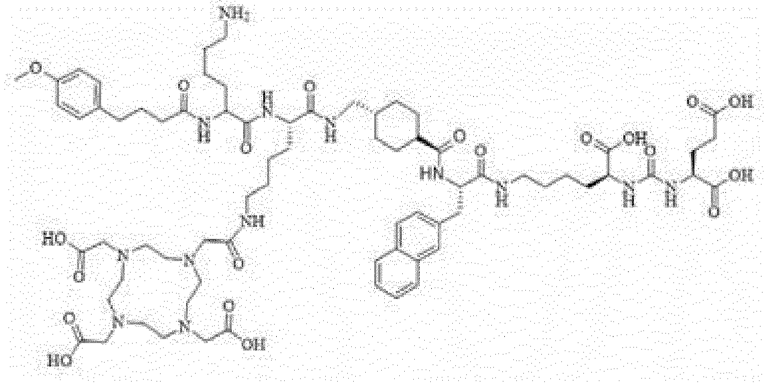
PSMA-D: 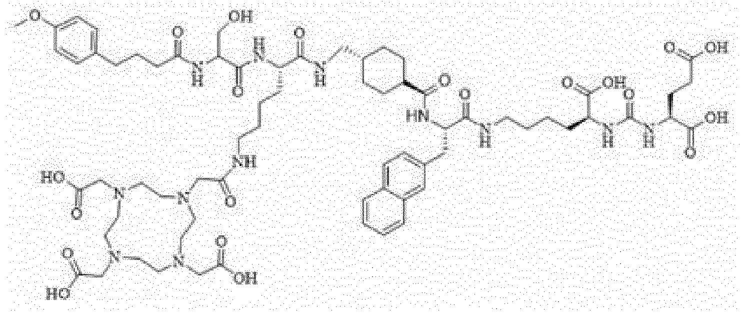
PSMA-E: 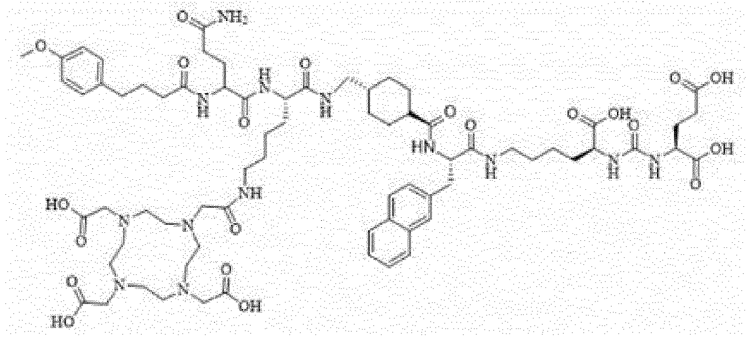
PSMA-F: 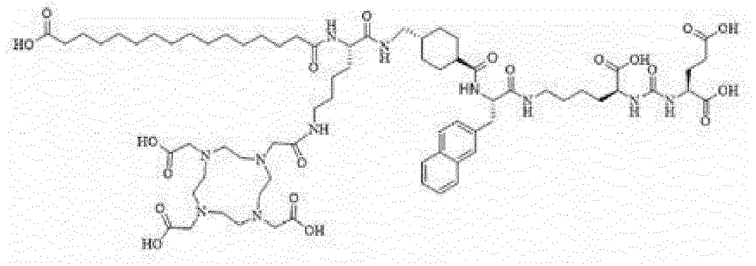
PSMA-G: 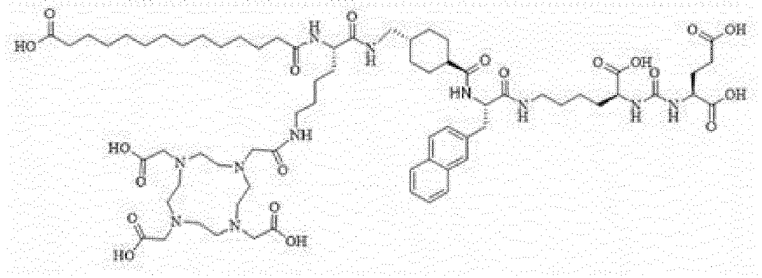
PSMA-H: 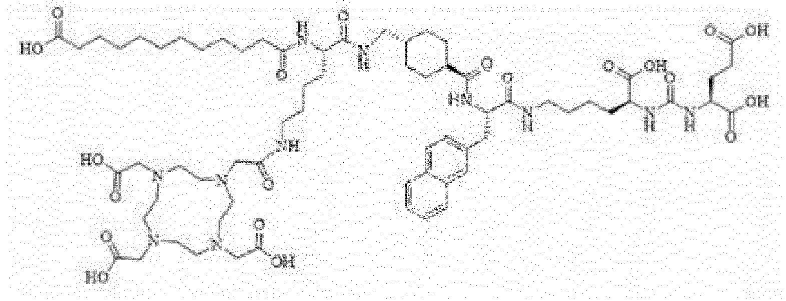
PSMA-I: 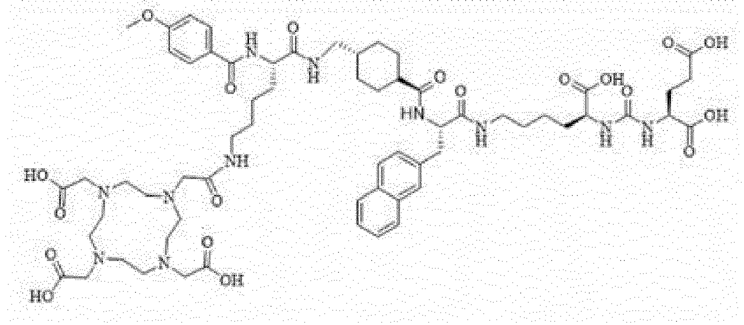
PSMA-J: 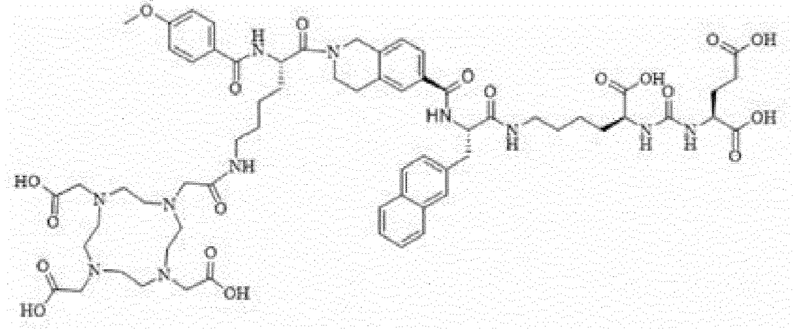
PSMA-K: 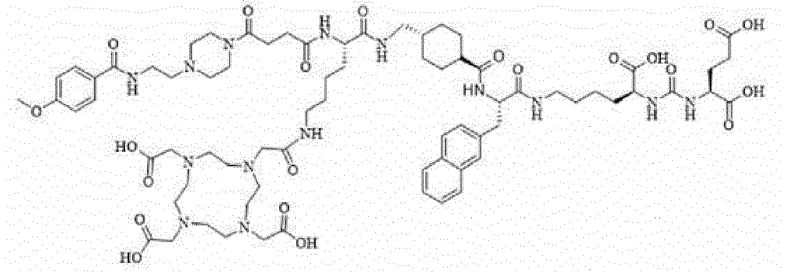
PSMA-L:

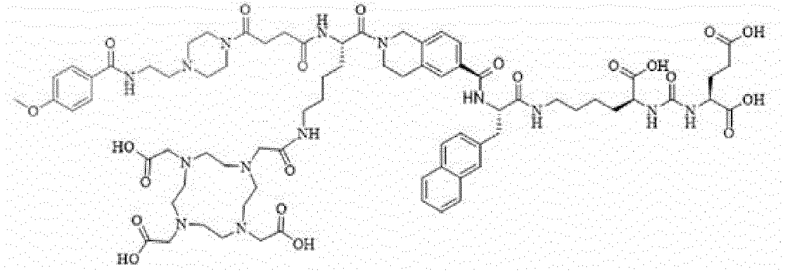

The raw materials of the present invention are all existing products, part of which are as shown as follows:

| **Reagent name** | **CAS number** | **Reagent name** | **CAS number** |
|---|---|---|---|
| DMF | 68-12-2 | Fmoc-Arg(Pbf)-OH | 154445-77-9 |
| DCM | 75-09-2 | Fmoc-His(Trt)-OH | 109425-51-6 |
| TEMP | 768-66-1 | DOTA | 137076-54-1 |
| DIEA | 7087-68-5 | Dde- Lys(F moc)-OH | 156648-40-7 |
| DIC | 693-13-0 | Fmoc-2-Nal-OH | 112883-43-9 |
| HOBT | 2592-95-2 | X6 | 167690-53-1 |
| TFA | 76-05-1 | p-methoxyphenylbutyric acid | 4521-28-2 |
| Hydrazine hydrate | 7803-57-8 | Fmoc-Gln(Trt)-OH | 132327-80-1 |
| H-Glu(OtBu)₂•HCl | 32677-01-3 | Fmoc-Ser(tBu)-OH | 1789-33-8 |
| fmoc-Lys(Dde)-OH | 150.629-67-7 | Fmoc-Lys(Boc)-OH | 71989-26-9 |
| Urea(DSC) | 74124-79-1 | | |

The above reagents are all conventionally purchased from commercial sources, and their purity is chemically pure (> 99.5%). Animal experiments used BALB/c mice (male, body weight 18-20 g) and SD rats (male, body weight 180-200 g), random grouping was performed, tumor bearing mouse models were conventionally constructed, and the animal experiments met the conventional animal experiment requirements. As a common knowledge, the crude product was purified by a reverse-phase high-performance liquid chromatograph, the chemical structure information of the product was characterized by MALDI-TOF mass spectrometry, and the purity was given by an analytical high-performance liquid chromatograph (Agela C18 4.6 × 250 mm, flow rate of 1 ml per min).

### Example 1

Step (1): 1 g of Fmoc-Lys(Dde)-Wang resin (1 eq) was weighed and put into a reactor as an insoluble solid phase carrier, and swollen with 30 mL of DCM for 1 h. The liquid in the reactor was removed by suction filtration, 30 mL of TEMP/DMF (30%, *i.e.,* a volume ratio of 3 : 10) was added to the resin, and a reaction was performed for 20 min to remove the Fmoc protecting group of the amino group. The liquid in the reactor was removed by suction filtration, washing was performed 6 times alternately with 30 mL of DMF and 30 mL of DCM, then 8 eq of Urea (DSC) and 16 eq of DIEA were added, and a reaction was performed for 120 min to form a Urea (DSC)-Lys (Dde)-Wang resin. The liquid in the reactor was removed by suction filtration, washing was performed 6 times alternately with 30 mL of DMF and 30 mL of DCM, and then 8 eq of H-Glu(OtBu)₂•HCl and 16 eq of DIEA were added for reacting with the Urea(DSC)-Lys(Dde)-Wang resin for 24 h to form a Glu(OtBu)₂-Urea-Lys(Dde)-Wang resin. The liquid in the reactor was removed by suction filtration, washing was performed 6 times alternately with 30 mL of DMF and 30 mL of DCM, 30 mL of a 3% hydrazine/DMF (volume ratio) solution was added, and a reaction was performed for 20 min to remove the Dde protecting group of the side chain amino group of Lys. The liquid in the reactor was removed by suction filtration, washing was performed 6 times alternately with 30 mL of DMF and 30 mL of DCM, then 8 eq of Fmoc-2-Nal-OH, 8 eq of HOBT and 8 eq of DIC were added, and a reaction was performed for 2 h to obtain a peptide chain. The liquid in the reactor was removed by suction filtration, washing was performed 6 times alternately with 30 mL of DMF and 30 mL of DCM, then 30 mL of TEMP/DMF (30%) was added, and a reaction was performed for 20 min to remove the Fmoc protecting group of Fmoc-2-Nal-OH. The liquid in the reactor was removed by suction filtration, washing was performed 6 times alternately with 30 mL of DMF and 30 mL of DCM, 8 eq of trans-4-(Fmoc-aminomethyl)cyclohexanecarboxylic acid (X6), 8 eq of HOBT and 8 eq of DIC were added to the resin, and a reaction was performed for 2 h to obtain a peptide chain Glu(OtBu)₂-Urea-Lys[(2-Nal)-X6-Fmoc]-Wang resin. The liquid in the reactor was removed by suction filtration, washing was performed 6 times alternately with 30 mL of DMF and 30 mL of DCM, then 30 mL of TEMP/DMF (30%) was added, and a reaction was performed for 20 min to remove the Fmoc protecting group of trans-4-(Fmoc-aminomethyl)cyclohexanecarboxylic acid. The liquid in the reactor was removed by suction filtration, washing was performed 6 times alternately with 30 mL of DMF and 30 mL of DCM, then 8 eq of Dde-Lys(Fmoc)-OH, 8 eq of HOBT and 8 eq of DIC were added, and a reaction was performed for 4 h to obtain a peptide chain Glu(OtBu)₂-Urea-Lys{(2-Nal)-X6-{Dde-Lys(Fmoc)]}-Wang resin.

Step (2): The peptide chain Glu(OtBu)₂-Urea-Lys{(2-Nal)-X6-{Dde-Lys(Fmoc)]}-Wang resin was washed 6 times alternately with 30 mL of DMF and 30 mL of DCM, then 30 mL of 30% TEMP/DMF (volume ratio) was added, and a reaction was performed for 20 min to remove the Fmoc protecting group. The liquid in the reactor was removed by suction filtration, washing was performed 6 times alternately with 30 mL of DMF and 30 mL of DCM, then 8 eq of DOTA, 8 eq of HOBT and 8 eq of DIC were added, and a reaction was performed for 4 h to obtain a peptide chain Glu(OtBu)₂-Urea-Lys{(2-Nal)-X6-[Dde-Lys(DOTA)]}-Wang resin. The liquid in the reactor was removed by suction filtration, washing was performed 6 times alternately with 30 mL of DMF and 30 mL of DCM, 30 mL of a 3% hydrazine/DMF (volume ratio) solution was added, and a reaction was performed for 20 min to remove the Dde protecting group. The liquid in the reactor was removed by suction filtration, washing was performed 6 times alternately with 30 mL of DMF and 30 mL of DCM, then 8 eq of amino acid raw material R, 8 eq of HOBT and 8 eq of DIC were added, and a reaction was performed for 2 h to obtain a peptide chain Glu(OtBu)₂-Urea-Lys[(2-Nal)-X6-Lys(DOTA)-R]. The liquid in the reactor was removed by suction filtration, washing was performed 6 times alternately with 30 mL of DMF and 30 mL of DCM, then 30 mL of TEMP/DMF (30%) was added, and a reaction was performed for 20 min to remove the Fmoc protecting group of R. The liquid in the reactor was removed by suction filtration, washing was performed 6 times alternately with 30 mL of DMF and 30 mL of DCM, then 8 eq of p-methoxyphenylbutyric acid (X8), 8 eq of HOBT and 8 eq of DIC were added, and a reaction was performed for 2 h to obtain a peptide chain. The liquid in the reactor was removed by suction filtration, washing was performed 6 times alternately with 30 mL of DMF and 30 mL of DCM. At this point, the peptide chain synthesis was completed, that is, the crude peptide with the protecting group was obtained: Glu(OtBu)₂-Urea-Lys[(2-Nal)-X6-Lys(DOTA)-R-X8]-Wang resin. Then washing was performed 6 times alternately with 30 mL of DMF and 30 mL of DCM, the resin was conventionally dried, then mixed with TFA and H₂O (a volume ratio of 95 : 5), a reaction was performed for 3 h, then the polypeptide was cleaved from the resin while removing the side chain protecting group, filtering was performed, the filtrate was added to ice absolute diethyl ether to precipitate the polypeptide, centrifuging was performed, the supernatant was poured out, then washing was performed with ice absolute diethyl ether, then centrifuging was performed, and the lower solid was dried to obtain the crude polypeptide. The crude product was purified by a reverse-phase high-performance liquid chromatograph, and the solvent was freeze-dried to obtain the pure polypeptide in a fluffy state, which was the product of the present invention, the ligand compound targeting PSMA antigen.

In the above step (2):
when the amino acid raw material R was Fmoc-Arg(Pbf)-OH, the product was compound PSMA-A with a molecular weight of 1502.78;
when the amino acid raw material R was Fmoc-His(Trt)-OH, the product was PSMA-B with a molecular weight of 1483.73;
when the amino acid raw material R was Fmoc-Lys(Boc)-OH, the product was PSMA-C with a molecular weight of 1474.76;
when the amino acid raw material R was Fmoc-Ser(tBu)-OH, the product was PSMA-D with a molecular weight of 1433.65;
when the amino acid raw material R was Fmoc-Gln(Trt)-OH, the product was PSMA-E with a molecular weight of 1474.2.

### Example 2

Step (1): 1 g of Fmoc-Lys(Dde)-Wang resin (1 eq) was weighed and put into a reactor as an insoluble solid phase carrier, and swollen with 30 mL of DCM for 1 h. The liquid in the reactor was removed by suction filtration, 30 mL of TEMP/DMF (30%, *i.e.,* a volume ratio of 3 : 10) was added to the resin, and a reaction was performed for 20 min to remove the Fmoc protecting group of the amino group. The liquid in the reactor was removed by suction filtration, washing was performed 6 times alternately with 30 mL of DMF and 30 mL of DCM, then 8 eq of Urea (DSC) and 16 eq of DIEA were added, and a reaction was performed for 120 min to form a Urea (DSC)-Lys (Dde)-Wang resin. The liquid in the reactor was removed by suction filtration, washing was performed 6 times alternately with 30 mL of DMF and 30 mL of DCM, and then 8 eq of H-Glu(OtBu)₂•HCl and 16 eq of DIEA were added for reacting with the Urea(DSC)-Lys(Dde)-Wang resin for 24 h to form a Glu(OtBu)₂-Urea-Lys(Dde)-Wang resin. The liquid in the reactor was removed by suction filtration, washing was performed 6 times alternately with 30 mL of DMF and 30 mL of DCM, 30 mL of a 3% hydrazine/DMF (volume ratio) solution was added, and a reaction was performed for 20 min to remove the Dde protecting group of the side chain amino group of Lys. The liquid in the reactor was removed by suction filtration, washing was performed 6 times alternately with 30 mL of DMF and 30 mL of DCM, then 8 eq of Fmoc-2-Nal-OH, 8 eq of HOBT and 8 eq of DIC were added, and a reaction was performed for 2 h to obtain a peptide chain. The liquid in the reactor was removed by suction filtration, washing was performed 6 times alternately with 30 mL of DMF and 30 mL of DCM, then 30 mL of TEMP/DMF (30%) was added, and a reaction was performed for 20 min to remove the Fmoc protecting group of Fmoc-2-Nal-OH. The liquid in the reactor was removed by suction filtration, washing was performed 6 times alternately with 30 mL of DMF and 30 mL of DCM, 8 eq of trans-4-(Fmoc-aminomethyl)cyclohexanecarboxylic acid (X6), 8 eq of HOBT and 8 eq of DIC were added to the resin, and a reaction was performed for 2 h to obtain a peptide chain Glu(OtBu)₂-Urea-Lys[(2-Nal)-X6-Fmoc]-Wang resin. The liquid in the reactor was removed by suction filtration, washing was performed 6 times alternately with 30 mL of DMF and 30 mL of DCM, then 30 mL of TEMP/DMF (30%) was added, and a reaction was performed for 20 min to remove the Fmoc protecting group of trans-4-(Fmoc-aminomethyl)cyclohexanecarboxylic acid. The liquid in the reactor was removed by suction filtration, washing was performed 6 times alternately with 30 mL of DMF and 30 mL of DCM, then 8 eq of Dde-Lys(Fmoc)-OH, 8 eq of HOBT and 8 eq of DIC were added, and a reaction was performed for 4 h to obtain a peptide chain Glu(OtBu)₂-Urea-Lys{(2-Nal)-X6-{Dde-Lys(Fmoc)]}-Wang resin.

Step (2): The peptide chain Glu(OtBu)₂-Urea-Lys{(2-Nal)-X6- {Dde-Lys(Fmoc)]}-Wang resin was washed 6 times alternately with 30 mL of DMF and 30 mL of DCM, then 30 mL of 30% TEMP/DMF (volume ratio) was added, and a reaction was performed for 20 min to remove the Fmoc protecting group of Dde-Lys(Fmoc). The liquid in the reactor was removed by suction filtration, washing was performed 6 times alternately with 30 mL of DMF and 30 mL of DCM, then 8 eq of DOTA, 8 eq of HOBT and 8 eq of DIC were added, and a reaction was performed for 4 h to obtain a peptide chain Glu(OtBu)₂-Urea-Lys{(2-Nal)-X6-[Dde-Lys(DOTA)]}-Wang resin. The liquid in the reactor was removed by suction filtration, washing was performed 6 times alternately with 30mL of DMF and 30 mL of DCM, 30 mL of a 3% hydrazine/DMF (volume ratio) solution was added, and a reaction was performed for 20 min to remove the Dde protecting group. The liquid in the reactor was removed by suction filtration, washing was performed 6 times alternately with 30 mL of DMF and 30 mL of DCM, then 8 eq of a precursor compound of R₁, 8 eq of HOBT and 8 eq of DIC were added, and a reaction was performed for 2 h to obtain a peptide chain Glu(OtBu)₂-Urea-Lys[(2-Nal)-X6-Lys(DOTA)-R₁]. The liquid in the reactor was removed by suction filtration, washing was performed 6 times alternately with 30 mL of DMF and 30 mL of DCM, the resin was conventionally dried, then mixed with TFA and H₂O (a volume ratio of 95 : 5), a reaction was performed for 3 h, then the polypeptide was cleaved from the resin while removing the side chain protecting group, filtering was performed, the filtrate was added to ice absolute diethyl ether to precipitate the polypeptide, centrifuging was performed, the supernatant was poured out, then washing was performed with ice absolute diethyl ether, then centrifuging was performed, and the lower solid was dried to obtain the crude polypeptide. The crude product was purified by a reverse-phase high-performance liquid chromatograph, and the solvent was freeze-dried to obtain the pure polypeptide in a fluffy state, which was the product of the present invention, the ligand compound targeting PSMA antigen.

In the above step (2):
when the precursor compound of R₁ was hexadecanedioic acid, the product was PSMA-F with a molecular weight of 1439.51 and a purity of 93.65%;
when the precursor compound of R₁ was tetradecanedioic acid, the product was PSMA-G with a molecular weight of 1411.88 and a purity of 95.85%;
when the precursor compound of R₁ was dodecanedioic acid, the product was PSMA-H with a molecular weight of 1383.22 and a purity of 97.32%;
when the precursor compound of R₁ was p-methoxybenzoic acid, the product was PSMA-I with a molecular weight of 1304.56 and a purity of 96.83%;
when the precursor compound of R₁ was X56, the product was PSMA-K with a molecular weight of 1516.53 and a purity of 95.42%; X56 had the following structure:

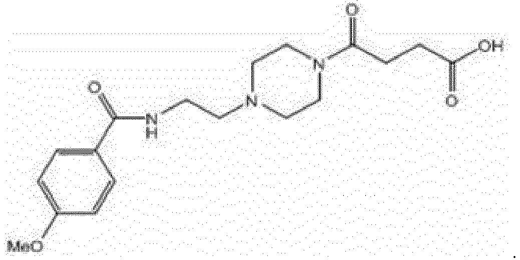

### Example 3

Step (1): 1 g of Fmoc-Lys(Dde)-Wang resin (1 eq) was weighed and put into a reactor as an insoluble solid phase carrier, and swollen with 30 mL of DCM for 1 h. The liquid in the reactor was removed by suction filtration, 30 mL of TEMP/DMF (30%, *i.e.,* a volume ratio of 3 : 10) was added to the resin, and a reaction was performed for 20 min to remove the Fmoc protecting group of the amino group. The liquid in the reactor was removed by suction filtration, washing was performed 6 times alternately with 30 mL of DMF and 30 mL of DCM, then 8 eq of Urea (DSC) and 16 eq of DIEA were added, and a reaction was performed for 120 min to form a Urea (DSC)-Lys (Dde)-Wang resin. The liquid in the reactor was removed by suction filtration, washing was performed 6 times alternately with 30 mL of DMF and 30 mL of DCM, and then 8 eq of H-Glu(OtBu)₂•HCl and 16 eq of DIEA were added for reacting with the Urea(DSC)-Lys(Dde)-Wang resin for 24 h to form a Glu(OtBu)₂-Urea-Lys(Dde)-Wang resin. The liquid in the reactor was removed by suction filtration, washing was performed 6 times alternately with 30 mL of DMF and 30 mL of DCM, 30 mL of a 3% hydrazine/DMF (volume ratio) solution was added, and a reaction was performed for 20 min to remove the Dde protecting group of the side chain amino group of Lys. The liquid in the reactor was removed by suction filtration, washing was performed 6 times alternately with 30 mL of DMF and 30 mL of DCM, then 8 eq of Fmoc-2-Nal-OH, 8 eq of HOBT and 8 eq of DIC were added, and a reaction was performed for 2 h to obtain a peptide chain Glu(OtBu)₂-Urea-Lys(2-Nal)-Wang resin. The liquid in the reactor was removed by suction filtration, washing was performed 6 times alternately with 30 mL of DMF and 30 mL of DCM, then 30 mL of TEMP/DMF (30%, *i.e.,* a volume ratio of 3 : 10) was added, and a reaction was performed for 20 min to remove the Fmoc protecting group of Fmoc-2-Nal-OH. The liquid in the reactor was removed by suction filtration, washing was performed 6 times alternately with 30 mL of DMF and 30 mL of DCM, 8 eq of X9, 8 eq of HOBT and 8 eq of DIC were added to the resin, and a reaction was performed for 2 h to obtain a peptide chain Glu(OtBu)2-Urea-Lys[(2-Nal)-X9-Fmoc]-Wang resin. The liquid in the reactor was removed by suction filtration, washing was performed 6 times alternately with 30 mL of DMF and 30 mL of DCM, then 30 mL of TEMP/DMF (30%) was added, and a reaction was performed for 20 min to remove the Fmoc protecting group of X9. The liquid in the reactor was removed by suction filtration, washing was performed 6 times alternately with 30 mL of DMF and 30 mL of DCM, then 8 eq of Dde-Lys(Fmoc)-OH, 8 eq of HOBT and 8 eq of DIC were added, and a reaction was performed for 4 h to obtain a peptide chain Glu(OtBu)₂-Urea-Lys { (2-Nal)-X9- { Dde-Lys(Fmoc)] }-Wang resin.

Step (2): The peptide chain Glu(OtBu)₂-Urea-Lys{(2-Nal)-X9-{Dde-Lys(Fmoc)]}-Wang resin was washed 6 times alternately with 30 mL of DMF and 30 mL of DCM, then 30 mL of 30% TEMP/DMF (volume ratio) was added, and a reaction was performed for 20 min to remove the Fmoc protecting group. The liquid in the reactor was removed by suction filtration, washing was performed 6 times alternately with 30 mL of DMF and 30 mL of DCM, then 8 eq of DOTA, 8 eq of HOBT and 8 eq of DIC were added, and a reaction was performed for 4 h to obtain a peptide chain Glu(OtBu)₂-Urea-Lys{(2-Nal)-X9-[Dde-Lys(DOTA)]}-Wang resin. The liquid in the reactor was removed by suction filtration, washing was performed 6 times alternately with 30 mL of DMF and 30 mL of DCM, 30 mL of a 3% hydrazine/DMF (volume ratio) solution was added, and a reaction was performed for 20 min to remove the Dde protecting group. The liquid in the reactor was removed by suction filtration, washing was performed 6 times alternately with 30 mL of DMF and 30 mL of DCM, then 8 eq of a precursor compound of R₁, 8 eq of HOBT and 8 eq of DIC were added, and a reaction was performed for 2 h to obtain a peptide chain Glu(OtBu)₂-Urea- Lys[(2-Nal)-X9-Lys(DOTA)-R₁]. The liquid in the reactor was removed by suction filtration, washing was performed 6 times alternately with 30 mL of DMF and 30 mL of DCM, the resin was conventionally dried, then mixed with TFA and H₂O (a volume ratio of 95 : 5), a reaction was performed for 3 h, then the polypeptide was cleaved from the resin while removing the side chain protecting group, filtering was performed, the filtrate was added to ice absolute diethyl ether to precipitate the polypeptide, centrifuging was performed, the supernatant was poured out, then washing was performed with ice absolute diethyl ether, then centrifuging was performed, and the lower solid was dried to obtain the crude polypeptide. The crude product was purified by a reverse-phase high-performance liquid chromatograph, and the solvent was freeze-dried to obtain the pure polypeptide in a fluffy state, which was the product of the present invention, the ligand compound targeting PSMA antigen. In the above step (2):
when the precursor compound of R₁ was p-methoxybenzoic acid, the product was PSMA-J with a molecular weight of 1309.29 and a purity of 96.00%;
when the precursor compound of R₁ was X56, the product was PSMA-L with a molecular weight of 1536.37 and a purity of 96.13%.

X56 had the following structure: 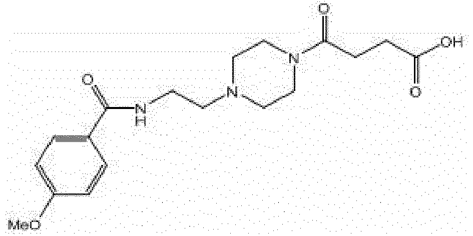 which could be prepared according to the following reaction:

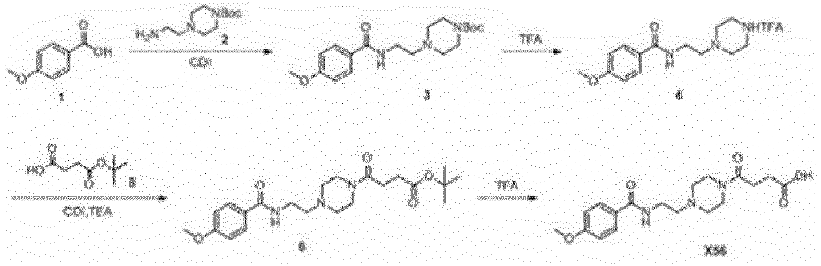

Compound 1 (1 g) was dissolved in 15 mL of dichloromethane, CDI (1.05 eq) was added, stirring was performed at room temperature for 1 h, then compound 2 (1 eq) was added, a reaction was performed for 2 h, and after the reaction was complete, separating was performed by column chromatography to obtain 2.2 g of compound 3. Compound 3 (2.2 g) was dissolved in 10 mL of dichloromethane, 5 mL of TFA was added, stirring was performed at room temperature for 1 h, and then the solvent was removed to obtain a crude compound 4, which was used directly in the next step. Compound 5 (1.05 g) was dissolved in 15 mL of dichloromethane, CDI (1.05 eq) was added, stirring was performed at room temperature for 1 h, then compound 4 (1 eq) and TEA (1.1 eq) were added, a reaction was performed for 2 h, and after the reaction was complete, separating was performed by column chromatography to obtain 1.4 g of compound 6. Compound 6 (1.4 g) was dissolved in 8 mL of dichloromethane, 4 mL of TFA was added, stirring was performed at room temperature for 1 h, then the solvent was removed to obtain a crude compound 7, and after preparative separation, 520 mg of a pure compound X56 was obtained.

X9 had the following structural formula: 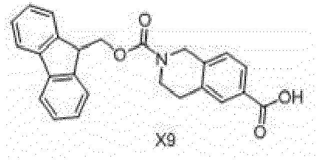 which could be prepared according to the following reaction:

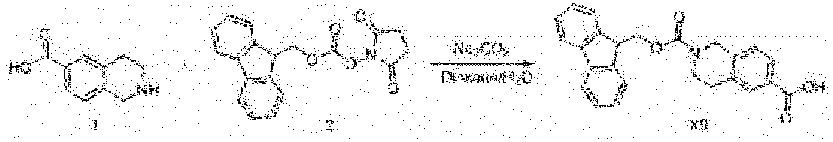

Compound 1 (1 g, 5.64 mmol) and Na₂CO₃ (1.2 g, 11.29 mmol) were added to 1,4-dioxane/H₂O, stirring was performed at room temperature for 30 min, then a solution of compound 2 (1.9 g, 5.64 mmol) in 1,4-dioxane was added dropwise to the system, the reaction was continued at room temperature for 2 h, and the completion of the reaction was tested by LCMS. The reaction liquid was concentrated to a small amount, and back extracted three times with ethyl acetate, the aqueous phase was retained, the pH of the aqueous phase was adjusted to weakly acidic with HCl (4 N), extracting was performed with ethyl acetate, purification was performed by pre-HPLC after concentration, and freeze-drying was performed to obtain 1.1 g of compound X9 as a white solid.

### Example 4 ¹⁷⁷Lu labeled precursor compound (chelate)

A metal bath reactor was opened and preheated to 95°C. The precursor (the ligand compound targeting PSMA antigen) was diluted to 100 ng/µL with a 0.5 M sodium acetate buffer (pH 4.5) to obtain a precursor solution. 37 MBq (1 mCi) of a ¹⁷⁷LuCl₃ solution (about 50 pmol) was taken, then 15 times equivalent of the precursor solution was added, the resulting mixture was supplemented to 50 µL with a sodium acetate buffer, and a reaction was performed at 95°C under 800 rpm for 30 min to obtain a ¹⁷⁷Lu labeled precursor compound in a solution form. TLC purity test was performed using 1% EDTA as a developing agent. If the purity was greater than 95%, no subsequent treatment was required. If the purity was less than 95%, subsequent purification was required: desalting was performed using a C18 column to remove the uncoordinated ¹⁷⁷Lu.

The radiochemical purity of the compounds PSMA-617, PSMA-A, PSMA-B, PSMA-C, PSMA-D, PSMA-E, PSMA-F, PSMA-G, PSMA-H, PSMA-I, PSMA-J, PSMA-K and PSMA-L labeled with ¹⁷⁷Lu all reached 95% or more.

### Example 5 SPECT/CT scanning and data processing

The ¹⁷⁷Lu labeled precursor compound was diluted to 7.4 MBq (200 µCi)/200 µL with normal saline, and each tumor bearing mouse was given about 7.4 MBq (200 µCi) of the drug via tail vein injection. SPECT/CT scanning was performed on the mice at 1, 4, 8, 24, 48 and 72 h after administration, respectively, with the mice under continuous anesthesia with isoflurane, lying prone on an examination bed. The acquisition method was static imaging 12 min SPECT, and medium resolution whole-body imaging CT. During the process, according to the recording table, the animal body weight, injection dose, injection time and residual dose were recorded, and the time of measuring the injection dose and the time of measuring the residual dose were recorded respectively. After scanning, the data were reconstructed, PMOD software was used to analyze and delineate each organ tissue and quantify the drug distribution therein, the images and data were saved, and the data were further subjected to statistical analysis.

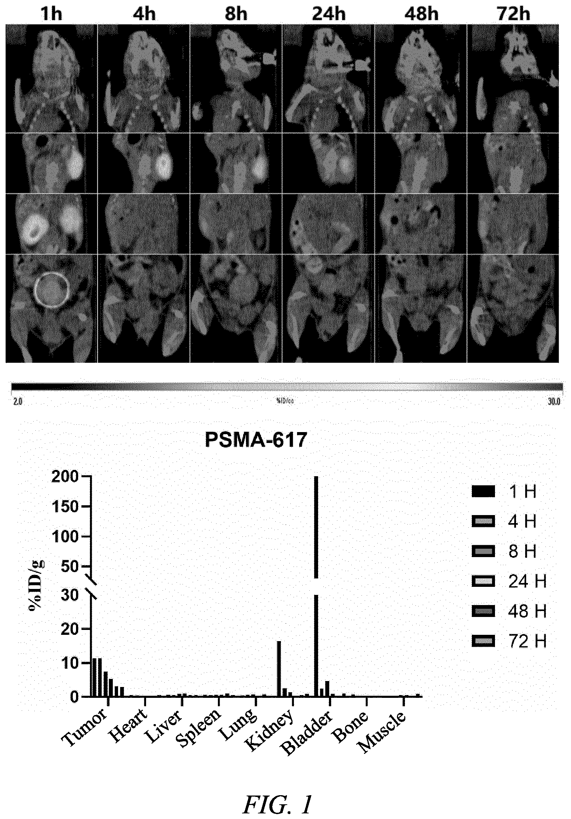

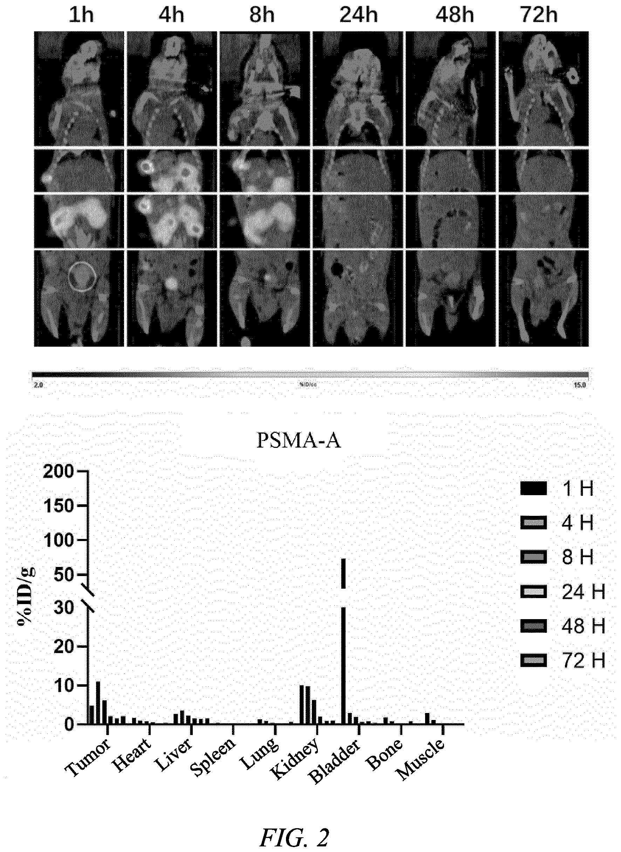

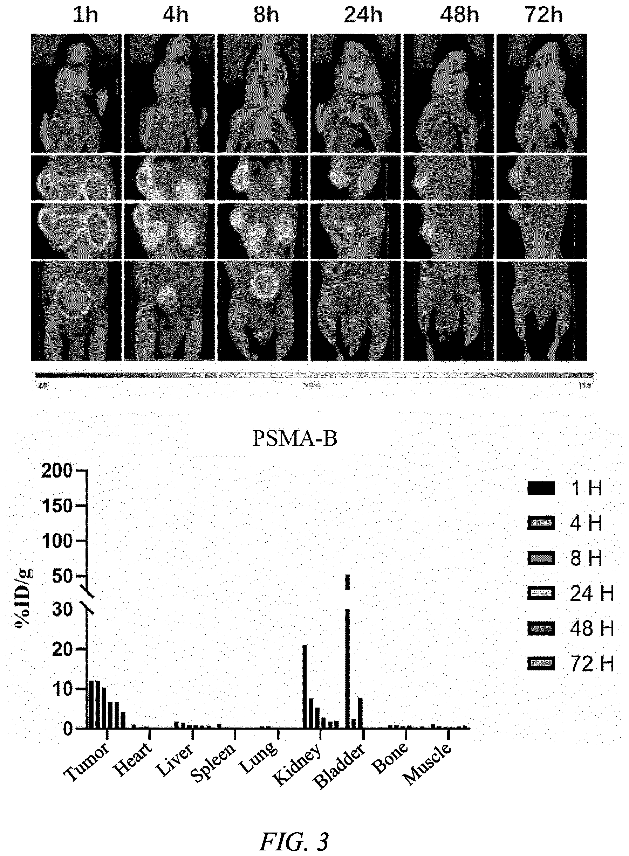

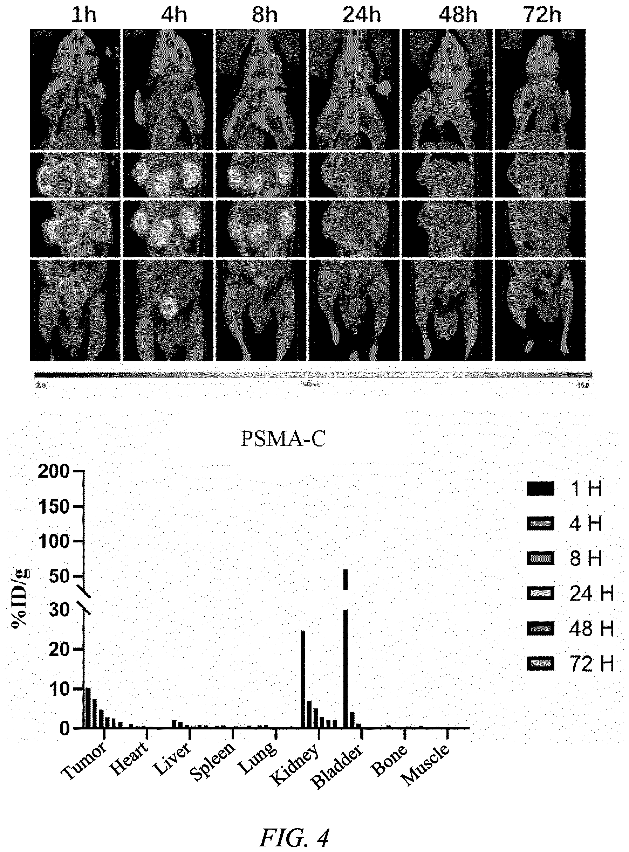

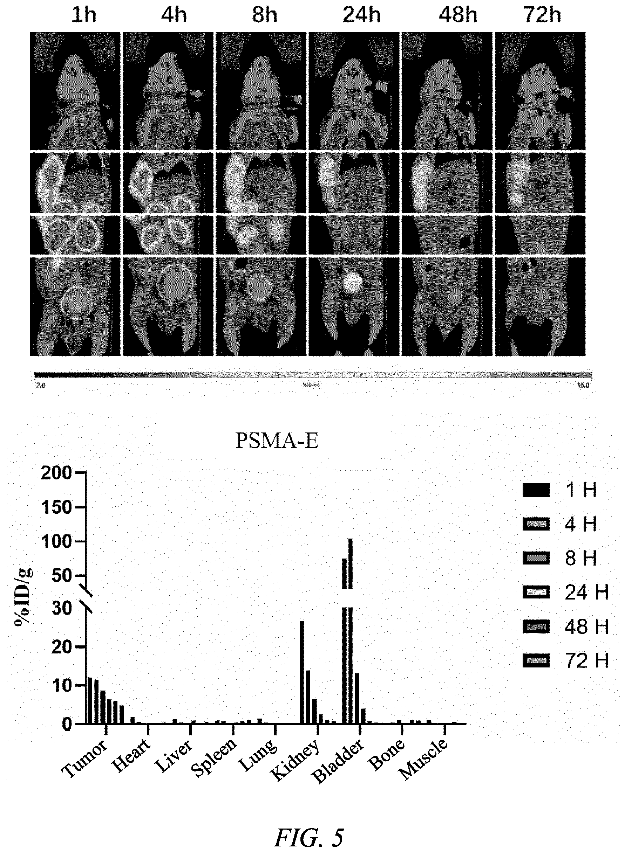

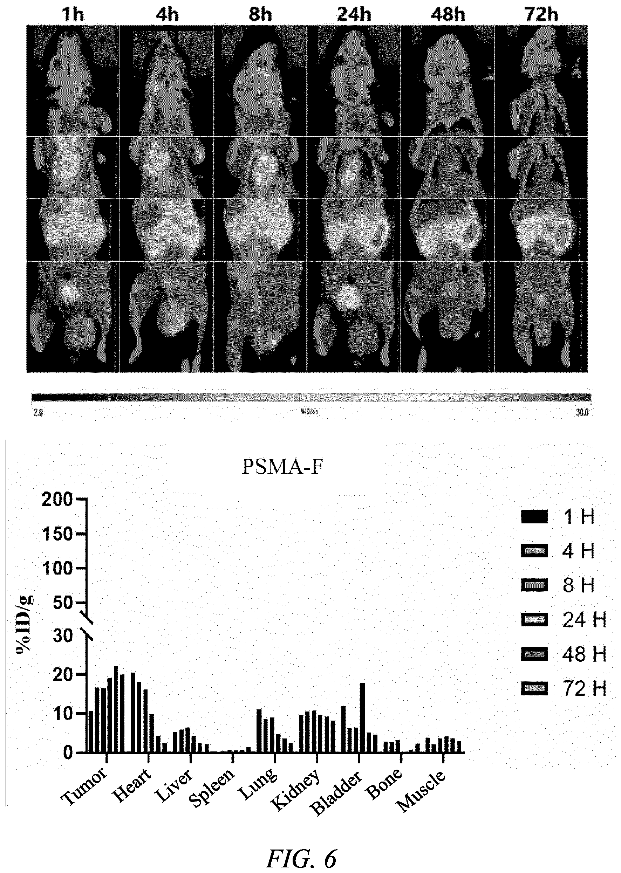

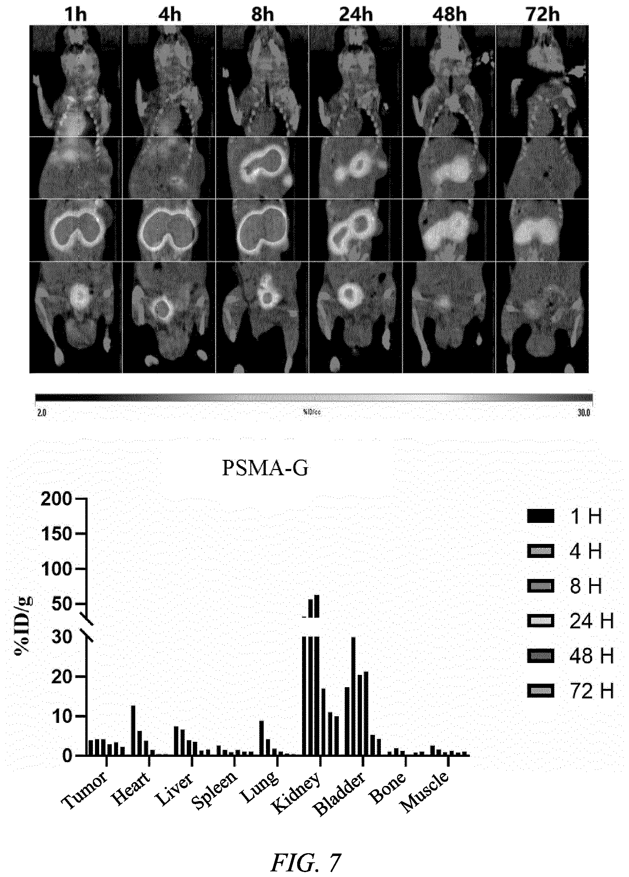

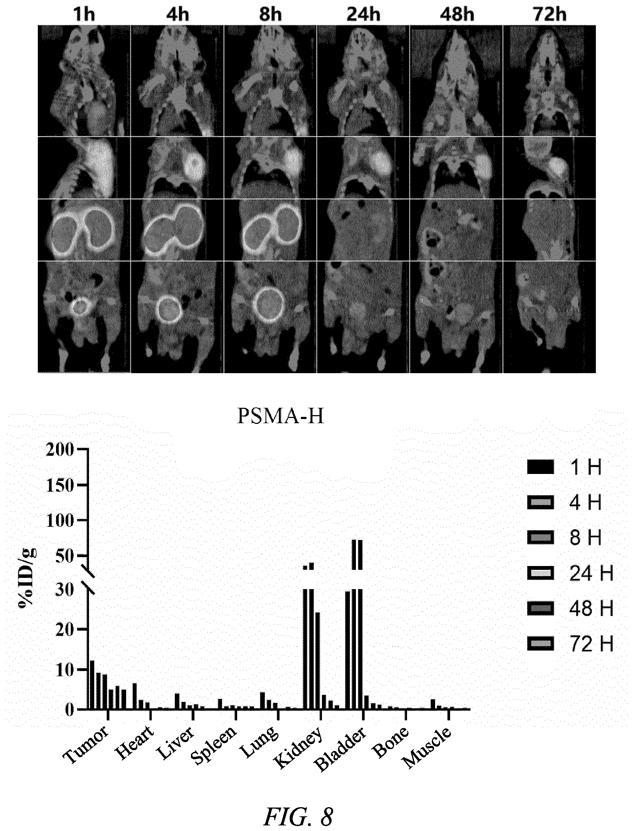

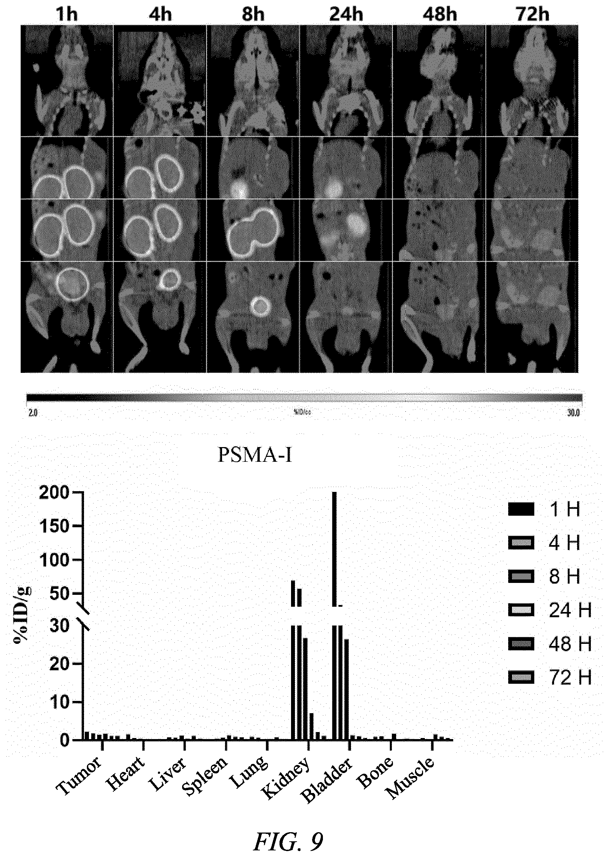

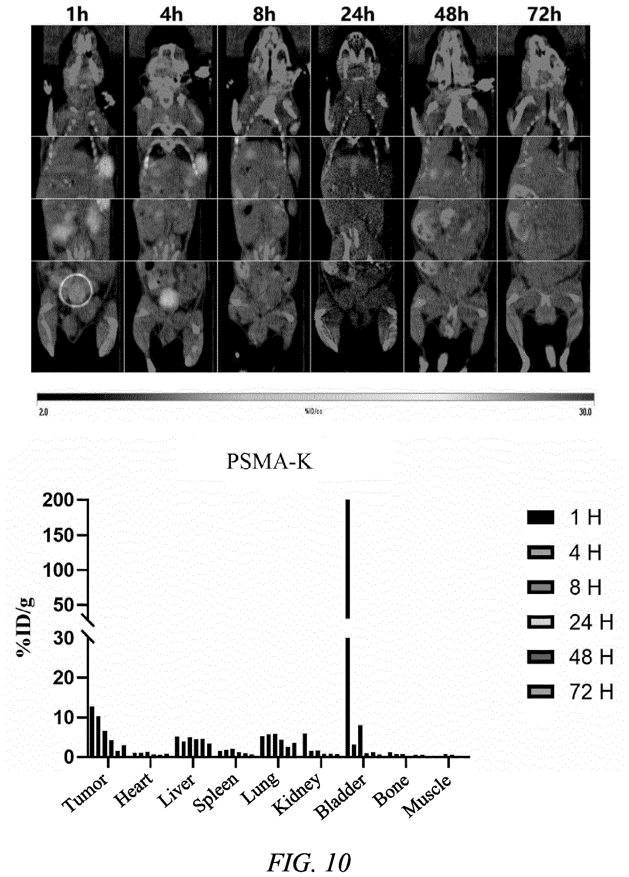

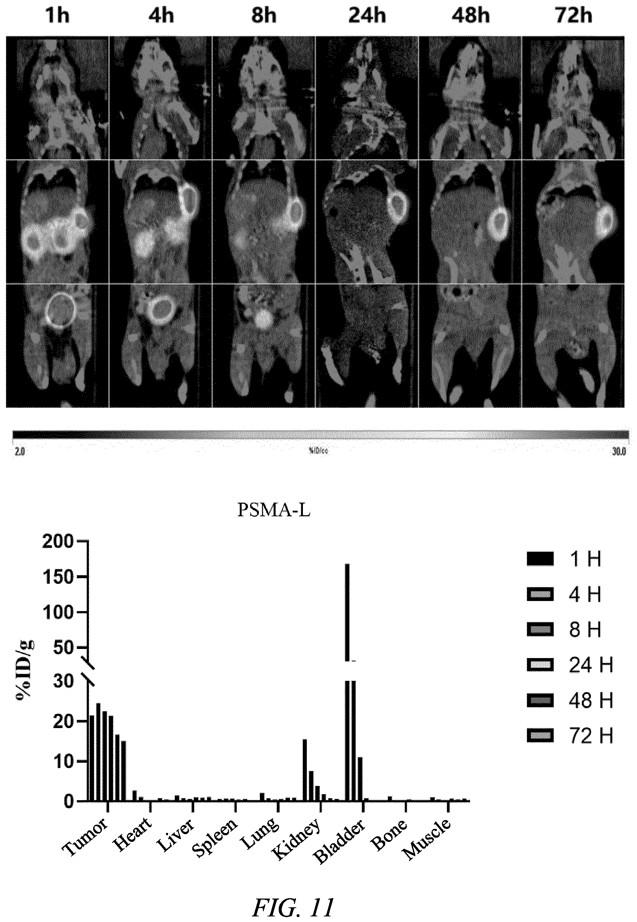

FIG. 1 to FIG. 11 showed the SPECT/CT scanning patterns of the compounds PSMA-617, PSMA-A, PSMA-B, PSMA-C, PSMA-E, PSMA-F, PSMA-G, PSMA-H, PSMA-I, PSMA-K and PSMA-L labeled with ¹⁷⁷Lu and the quantitative value of drug distribution in each tissue. Drug exposure in each tissue was calculated (Table 1, where the values involving PSMA-D were the predicted values). In contrast to PSMA-617, it could be seen that the ligand compound of the present invention could improve the exposure in the LNCaP tumor and the tissues, and in particular PSMA-L exposure in the normal tissues was kept at a lower level, so that PSMA-L could greatly increase the drug exposure in the LNCaP tumor while maintaining the low drug exposure in the normal tissues, which was a more potent compound than PSMA-617.

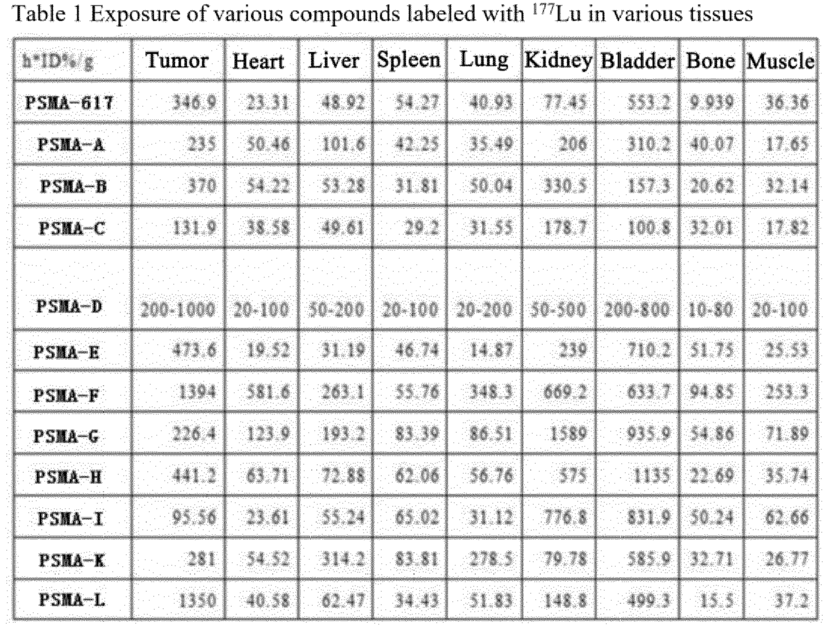

### Example 6 Cell binding ability

PSMA positive cells LNCaP were proliferated and cultured, digested, and then seeded in a 24-well plate at a density of 10⁵ cells/well. After cell adherence, the culture medium was aspirated, and the ¹⁷⁷Lu labeled compound containing a gradient radioactive count and positive control PSMA-617 were added. The highest concentration was 5 × 10⁶ cpm/0.5 mL, and gradient dilution was performed according to a dilution ratio of 1/3. After the labeled compound was added, the cells were incubated at 37°C for 1 h, the culture medium was aspirated, the cells were washed 3 times with PBS, and 0.2 mL of a 1 M NaOH solution was added for lysing the cells. The lysate was pipetted into a radioimmunoassay tube, and the radioactive count of each sample tube was tested by a γ counter. The data were analyzed, and the binding ability of the candidate compound to PSMA positive cells and the binding ability of the positive control compound PSMA-617 to PSMA positive cells were compared.

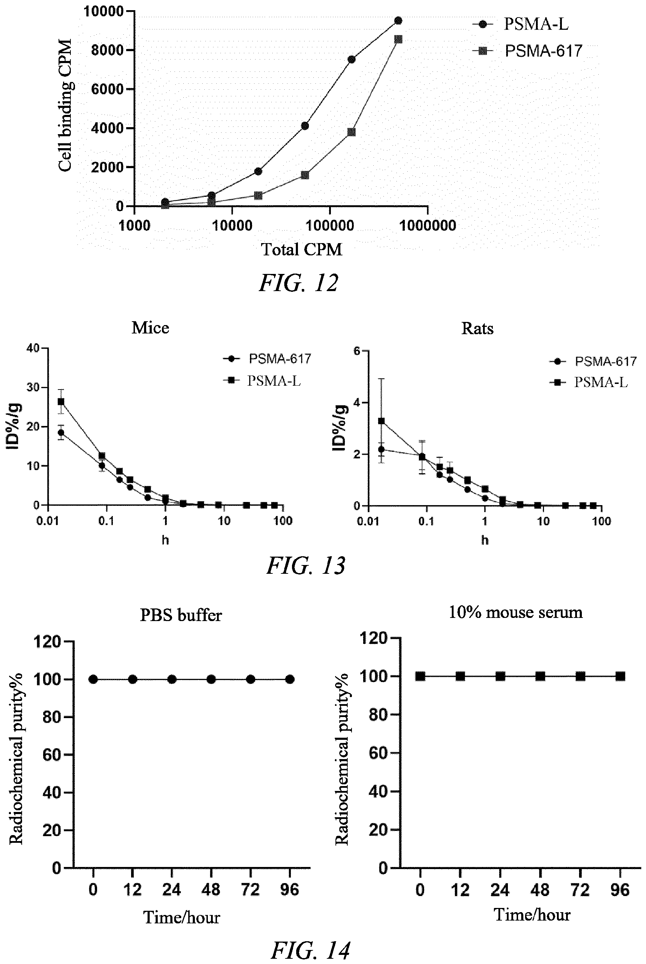

FIG. 12 showed the binding of ¹⁷⁷Lu-PSMA-617 to LNCaP cells and the binding of ¹⁷⁷Lu-PSMA-L to LNCaP cells. It could be seen that the binding ability of ¹⁷⁷Lu-PSMA-L to LNCaP cells was stronger than that of ¹⁷⁷Lu-PSMA-617 to LNCaP cells, indicating that ¹⁷⁷Lu-PSMA-L had higher antigen affinity than ¹⁷⁷Lu-PSMA-617.

### Example 7 Pharmacokinetics

BALB/c mice, male, body weight 18-20 g, SD rats, male, body weight 180-200 g. Random grouping was performed (3 animals in each group). After anesthesia with isoflurane, tail vein injection of 1.85 MBq (50 µCi) of the ¹⁷⁷Lu labeled compounds was performed respectively. 20 µL of blood was collected from the orbits at 1 min, 5 min, 10 min, 15 min, 0.5 h, 1 h, 2 h, 4 h, 8 h, 24 h and 72 h after administration into a pre-weighed centrifugal tube, respectively. The radioactive count of the blood in the centrifugal tube and the weight of the centrifugal tube having the blood were measured, and the radioactive count in the per unit weight of blood as a percentage of the amount administered was calculated. Drug-time curve diagrams were plotted, and the exposure of the compound of the present invention in blood and the exposure of the positive control compound PSMA-617 in blood were calculated.

FIG. 13 showed the pharmacokinetic profiles of ¹⁷⁷Lu-PSMA-617 and ¹⁷⁷Lu-PSMA-L in mice and rats. It could be seen that ¹⁷⁷Lu-PSMA-L was metabolized more slowly than ¹⁷⁷Lu-PSMA-617 in both mice and rats, and had a longer blood half-life and higher drug exposure. Moreover, this higher blood drug concentration was only maintained 1 h after administration, and then ¹⁷⁷Lu-PSMA-L was also substantially cleared out of the blood, thereby increasing the action concentration and action time of the drug to the tumor without increasing toxic and side effects on normal tissues.

### Example 8 Stability of radiochemical purity

200 µCi of the compound labeled with ¹⁷⁷Lu was taken, and mixed with 0.5 mL of PBS and 10% mouse serum, respectively. Sampling was performed at 12 h, 24 h, 48 h, 72 h and 96 h after mixing, respectively, and the radiochemical purity of the samples was tested by TLC to study the stability of radiochemical purity of the compounds.

FIG. 14 showed the stability of radiochemical purity of ¹⁷⁷Lu-PSMA-L in PBS buffer and the stability of radiochemical purity of ¹⁷⁷Lu-PSMA-L in 10% mouse serum. It could be seen that the stability of radiochemical purity of ¹⁷⁷Lu-PSMA-L in PBS buffer and the stability of radiochemical purity of ¹⁷⁷Lu-PSMA-L in 10% mouse serum were both maintained at 100% within 96 hours, which were very stable. In view of the results of scanning, it was indicated that ¹⁷⁷Lu was coordinated with PSMA-L very stably, and did not fall off *in vitro* and *in vivo,* which was very beneficial for the production, transportation and preservation of the drug, and diagnosis and treatment in the later stage.

### Example 9 Hematotoxicity

Mice were randomly grouped according to their body weights, (5 mice in each group), each mouse was given 7.4 MBq (200 µCi) or 14.8 MBq (400 µCi) of the compound labeled with ¹⁷⁷Lu via tail vein injection, and the control group was given PBS. After 7 days, the animals were anesthetized, 1 mL or more of blood was collected and placed in an anti coagulation tube, and the content of leukocytes, hemoglobin and platelets in the blood was determined by a mouse-use blood biochemical analyzer. The control group and the administration group were compared to understand the degree of bone marrow suppression by the drug.

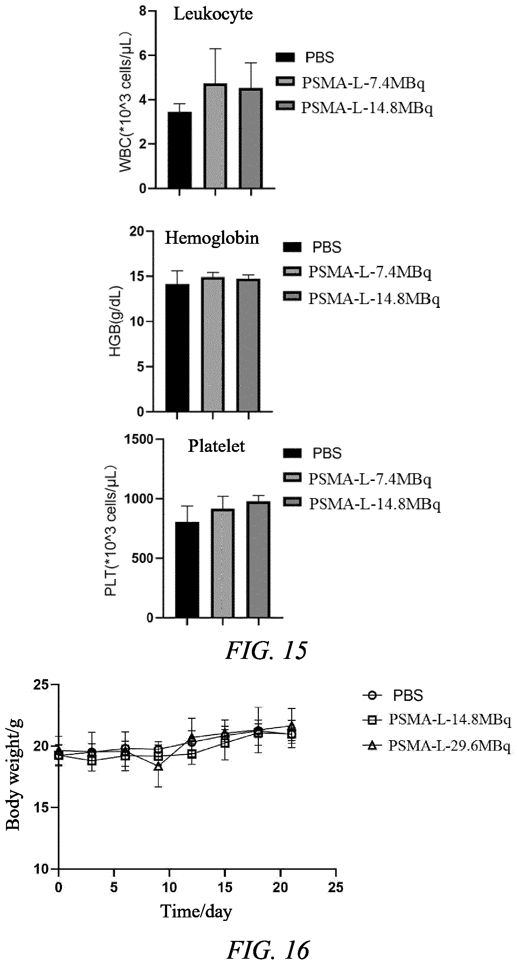

FIG. 15 showed the effect of ¹⁷⁷Lu-PSMA-L on the leukocytes, hemoglobin, platelets in mouse blood. It could be seen that at the administration doses of 7.4 MBq (200 µCi) and 14.8 MBq (400 µCi), there was no significant difference in the content of leukocytes, hemoglobin and platelets in the blood of the mice between the administration group and the control PBS group, indicating that ¹⁷⁷Lu-PSMA-L had a very low hematotoxicity and weak bone marrow suppression. This was related to the pharmacokinetic properties of ¹⁷⁷Lu-PSMA-L, with ¹⁷⁷Lu-PSMA- L maintaining a high blood drug level only 1 h after administration and then being rapidly cleared from the blood, thereby reducing the damage to normal cells in the blood.

### Example 10 Dose tolerance

Mice were randomly grouped according to their body weights, (5 mice in each group), each mouse was given 14.8 MBq (400 µCi) or 29.6 MBq (800 µCi) of the compound labeled with ¹⁷⁷Lu via tail vein injection, and the control group was given a solvent. The body weights of the mice were measured every 3 days after administration, and the death of the mice was recorded. The control group and the administration group were compared to understand the maximum tolerated dose of the drug in the mice.

FIG. 16 showed the effect of ¹⁷⁷Lu-PSMA-L on the body weight of mice at different administration doses. It could be seen that at either the administration dose of 14.8 MBq (400 µCi) or the administration dose of 29.6 MBq (800 µCi), there was no significant decrease in the body weight of the mice, indicating that there was a very high dose tolerance of ¹⁷⁷Lu-PSMA-L in the mice and that the safety of ¹⁷⁷Lu-PSMA-L was good.

### Example 11 Inhibition on tumor

PSMA positive LNCaP cells were proliferated and harvested, and then inoculated subcutaneously in mice at an amount of 1 × 10⁷/mouse. When the tumors grew to 200-400 mm³, the mice were randomly grouped according to the tumor size (3-4 mice in each group), and were given the compound (400 µCi) of the present invention labeled with ¹⁷⁷Lu or the positive control compound PSMA-617 labeled with ¹⁷⁷Lu via tail vein injection, respectively. The tumor sizes and body weights of the mice were measured every 3 days after administration, and the death of the mice was recorded. The control group and the administration group were compared to understand the inhibition on LNCaP tumors by different drugs. On day 6 after administration, SPECT/CT scanning was performed on the administration group to understand the exposure of different drugs at the tumor sites during the treatment.

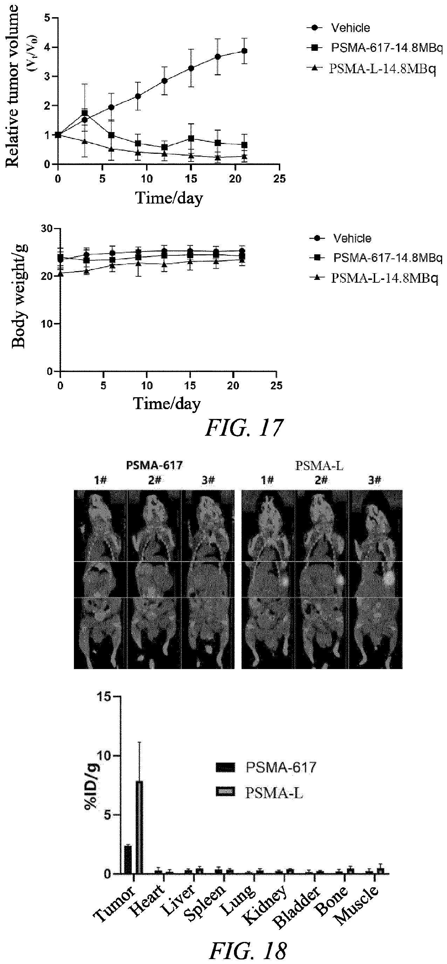

FIG. 17 showed the inhibitory effects of ¹⁷⁷Lu-PSMA-617 and ¹⁷⁷Lu-PSMA-L on the tumor in LNCaP tumor bearing mice and the change in the body weight of animals during the treatment. It could be seen that ¹⁷⁷Lu-PSMA-L had a stronger inhibitory effect on the tumor in the LNCaP tumor bearing mice than ¹⁷⁷Lu-PSMA-617. During the treatment, there was no significant difference in the body weight of the animals in each group, indicating that the mice could tolerate both 14.8 MBq (400 µCi) of ¹⁷⁷Lu-PSMA-617 and 14.8 MBq (400 µCi) of ¹⁷⁷Lu-PSMA-L.

FIG. 18 showed the radioactivity activity at the tumor site and the quantitative value of the radioactivity activity at the tumor site during the inhibition on the tumor in the LNCaP tumor bearing mice by ¹⁷⁷Lu-PSMA-617 and ¹⁷⁷Lu-PSMA-L. It could be seen that on day 6 after administration, the exposure of ¹⁷⁷Lu-PSMA-L at the tumor site was higher than that of ¹⁷⁷Lu-PSMA-617, which was also consistent with the results of the inhibitory effects on the tumor by the drugs.

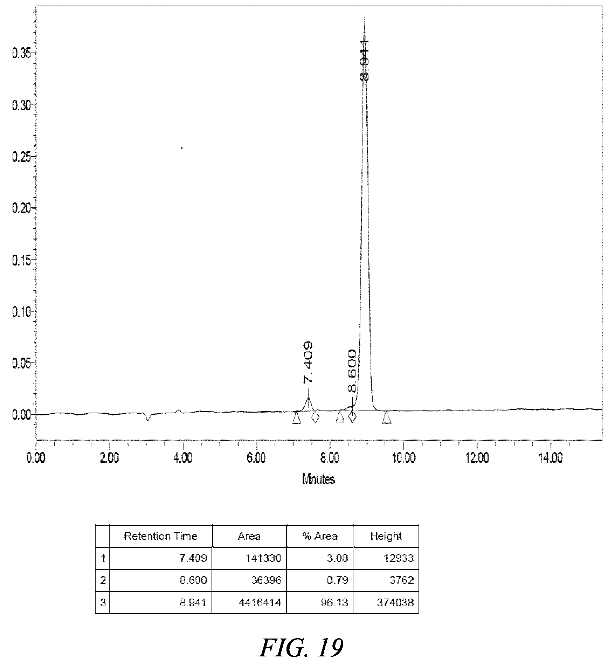

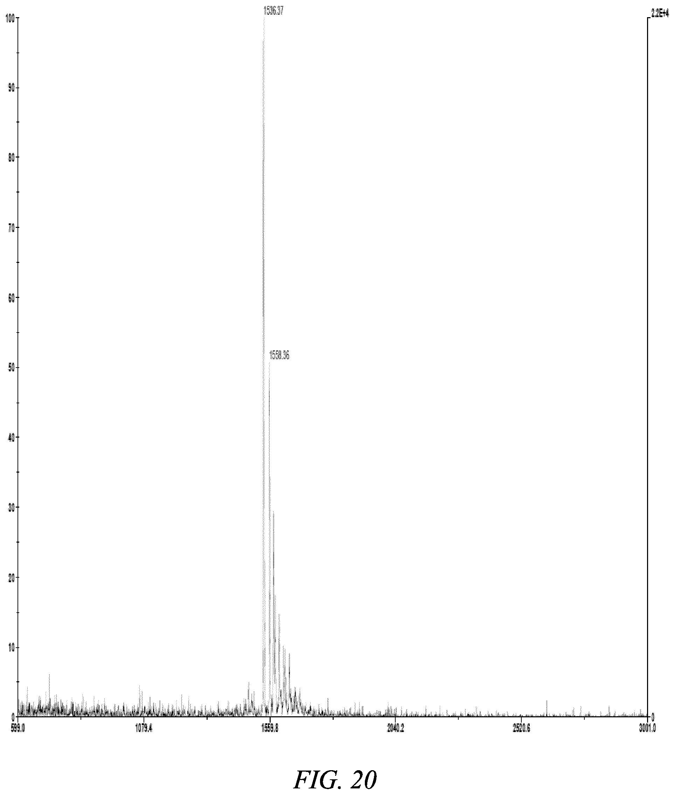

FIG. 19 showed the liquid chromatogram of the above compound PSMA-L; FIG. 20 showed the mass spectrum of the above compound PSMA-L;

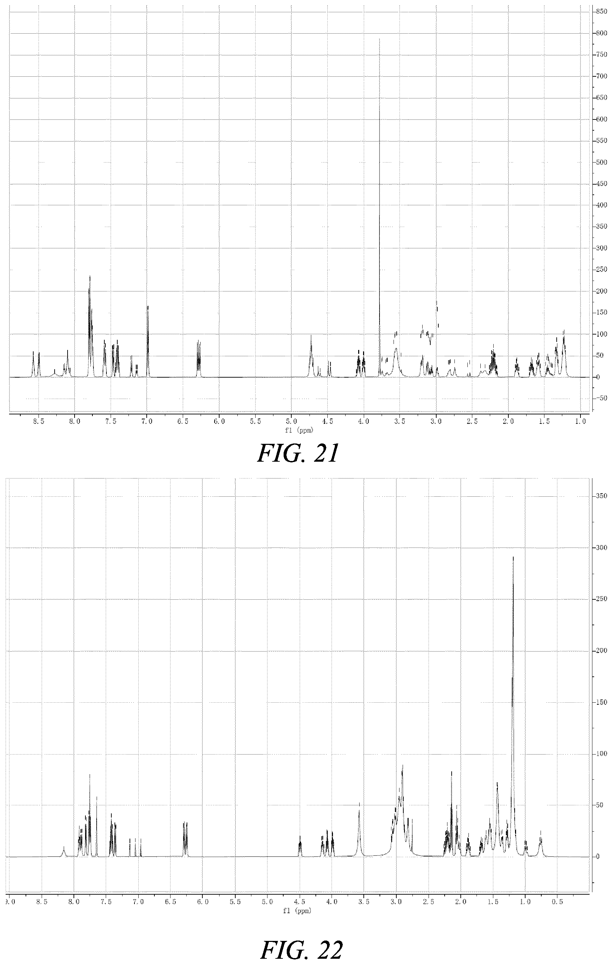

FIG. 21 showed the nuclear magnetic resonance pattern of the above compound PSMA-L, ¹H NMR (600 MHz, dmso);

FIG. 22 showed the nuclear magnetic resonance pattern of the above compound PSMA-H, ¹H NMR (600 MHz, dmso).

Prostate cancer is cancer that occurs in the prostate. The prostate is a walnut-shaped gland in the pelvis of men. In castration-resistant prostate cancer (CRPC), the tumor still shows signs of growth despite the use of testosterone-lowering hormone therapy. Radioligand therapy combines a targeting compound that can bind to a marker expressed by a tumor with a radioisotope, and can cause DNA injury and inhibit tumor growth and replication. This therapy enables targeted delivery of radiation to tumors while limiting damage to surrounding normal tissues. ¹⁷⁷Lu-PSMA-617 is a PSMA-targeted radioligand therapy for metastatic castration-resistant prostate cancer (mCRPC), and has entered clinical study. In contrast, the chelate composed of the ligand compound of the present invention and the radioactive metal has a lower toxicity to normal tissues, higher exposure dose at the tumor site, longer half-life in blood, better antigen affinity, better inhibitory effect on tumor, good stability of radiochemical purity and good safety *in vivo,* and has high application value in the diagnosis and treatment of prostate cancer.

## Claims

1. A ligand compound targeting a PSMA antigen or a pharmaceutically acceptable salt or ester thereof or a solvate thereof, **characterized in that** the ligand compound has the following chemical structural formula: 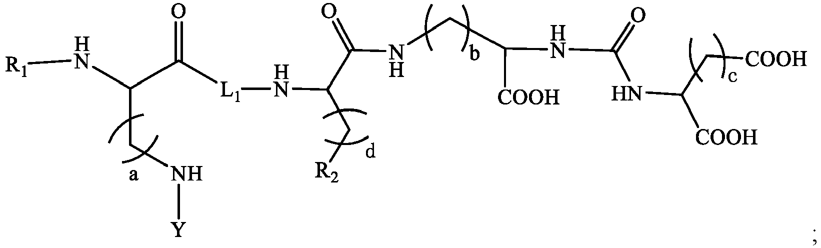
wherein a and b are each independently any integer of 1 to 8, and c and d are each independently any integer of 1 to 4; preferably, a and b are each independently any integer of 2 to 6, and c and d are each independently any integer of 1 to 3; more preferably, a and b are 4, c is 2, and d is 1;
L₁ has any one of the following chemical structural formulas: 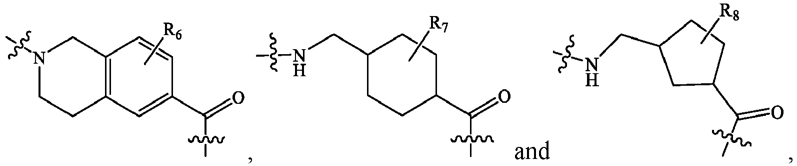 and preferably, L₁ is linked to other fragments in the structural formula of the ligand compound via amide bonds;
wherein R₆, R₇, and R₈ are each independently any one of hydrogen, alkyl, alkoxy, and halogen;
R₁ has any one of the following chemical structural formulas: 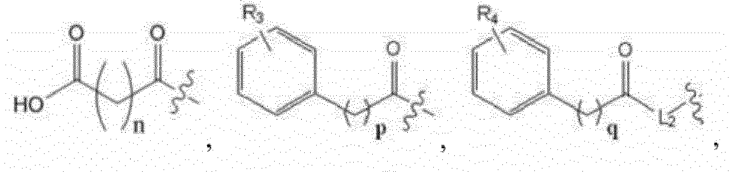 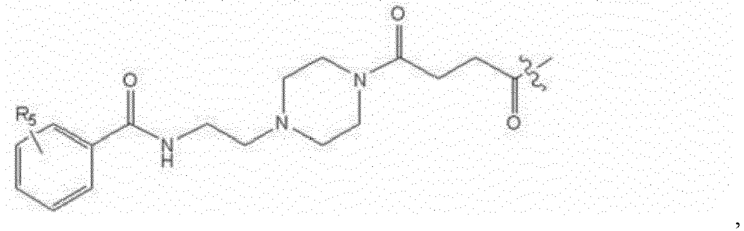
wherein n is any integer of 2 to 20; R₃, R₄, and R₅ are each independently any one of hydrogen, alkyl, alkoxy, halogen, cycloalkyl, heterocyclyl, and aryl; p is any integer of 0 to 8; q is any integer of 1 to 10; L₂ is an amino acid linker structure;
R₂ is aryl or heteroaryl, preferably aryl; and Y is a radioactive metal chelator group.

2. The ligand compound or the pharmaceutically acceptable salt or ester thereof or the solvate thereof according to claim 1, **characterized in that** the ligand compound has the following chemical structural formula: 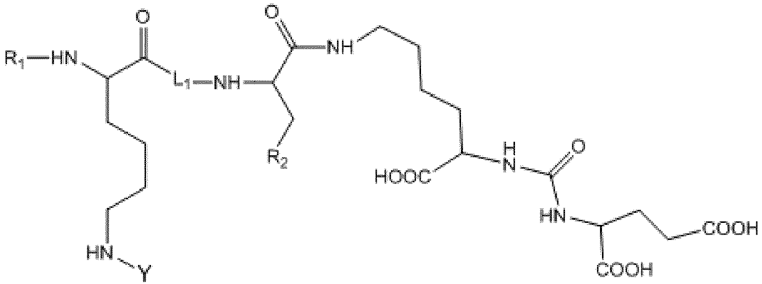
wherein L₁ has any one of the following chemical structural formulas: 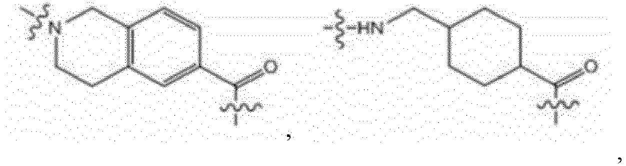 and preferably, L₁ is linked to other fragments in the structural formula of the ligand compound via amide bonds;
R₁, R₂ and Y are as defined in claim 1.

3. The ligand compound or the pharmaceutically acceptable salt or ester thereof or the solvate thereof according to claim 1 or 2, **characterized in that** the radioactive metal chelator is any one of DOTA or a derivative thereof, TETA or a derivative thereof, SarAR or a derivative thereof, NOTA or a derivative thereof, TRAP or a derivative thereof, HBED or a derivative thereof, 2,3-HOPO or a derivative thereof, PCTA or a derivative thereof, DFO or a derivative thereof, DTPA or a derivative thereof, OCTAPA or a derivative thereof, and H₂-MACROPA or a derivative thereof.

4. A DOTA-based ligand compound targeting a PSMA antigen or a pharmaceutically acceptable salt or ester thereof or a solvate thereof, **characterized in that** the ligand compound has the following chemical structural formula: 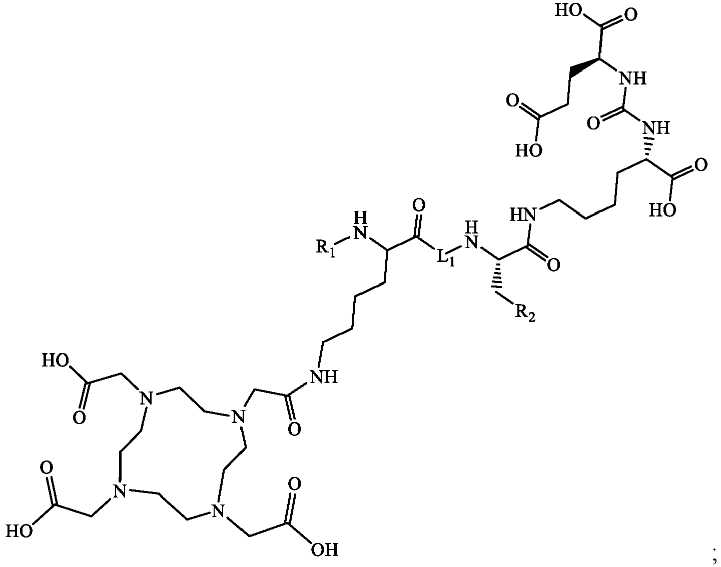
wherein L₁ has any one of the following chemical structural formulas: 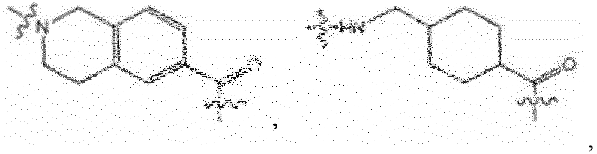 and preferably, L₁ is linked to other fragments in the structural formula of the ligand compound via amide bonds;
R₁ has any one of the following chemical structural formulas: 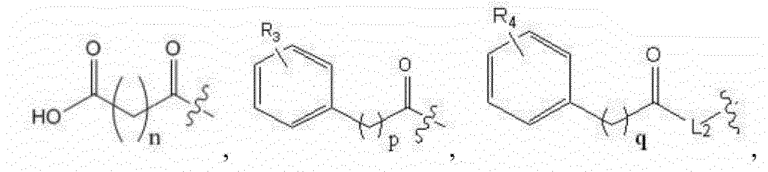 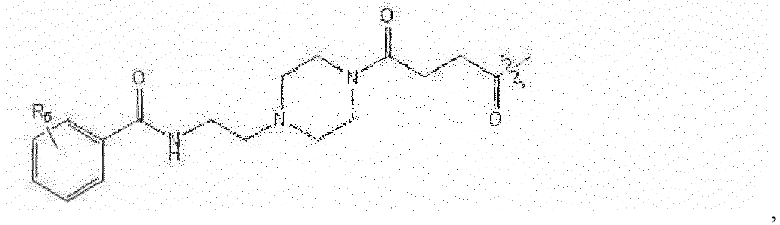
wherein n is any integer of 2 to 20; R₃, R₄, and R₅ are each independently any one of hydrogen, alkyl, alkoxy, halogen, cycloalkyl, heterocyclyl, and aryl; p is any integer of 0 to 8; q is any integer of 1 to 10; L₂ is an amino acid linker structure;
and R₂ is aryl or heteroaryl, preferably aryl, and more preferably naphthyl.

5. The ligand compound or the pharmaceutically acceptable salt or ester thereof or the solvate thereof according to claim 4, **characterized in that** the ligand compound has the following chemical structural formula: 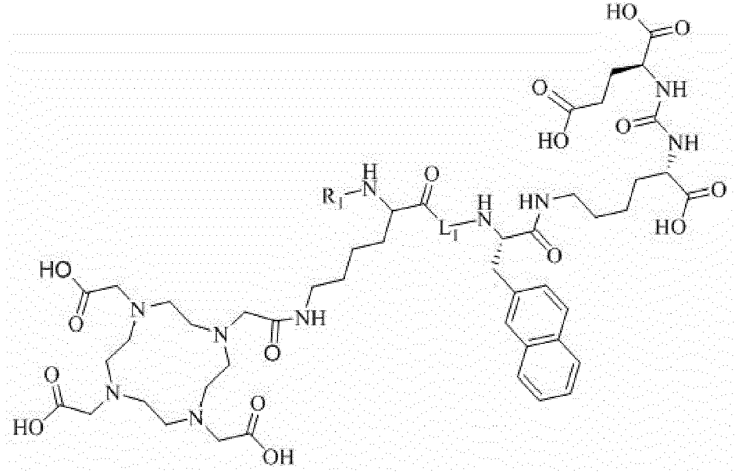 wherein R₁ and L₁ are as defined in claim 4.

6. The ligand compound or the pharmaceutically acceptable salt or ester thereof or the solvate thereof according to any one of claims 1-5, **characterized in that** the n is any integer of 6 to 16; the alkoxy is alkoxy containing 1 to 6 carbon atoms; the alkyl is alkyl containing 1 to 6 carbon atoms; the q is any integer of 2 to 5; the p is any integer of 0 to 4;
preferably, the n is any integer of 8 to 14; the alkoxy is alkoxy containing 1 to 3 carbon atoms; the alkyl is alkyl containing 1 to 3 carbon atoms; the p is 0 or 1; and the q is 2 or 3.

7. The ligand compound or the pharmaceutically acceptable salt or ester thereof or the solvate thereof according to any one of claims 1-5, **characterized in that** the aryl is phenyl, naphthyl or anthryl.

8. The ligand compound or the pharmaceutically acceptable salt or ester thereof or the solvate thereof according to any one of claims 1-5, **characterized in that** the amino acid in the amino acid linker structure is arginine, serine, histidine, lysine, glycine or glutamine.

9. The ligand compound or the pharmaceutically acceptable salt or ester thereof or the solvate thereof according to any one of claims 1-5, **characterized in that** the R₁ has the following chemical structural formula: 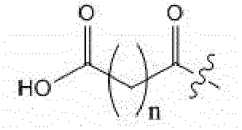
wherein n is any integer of 6 to 16;
preferably, n is any integer of 8 to 14.

10. The ligand compound or the pharmaceutically acceptable salt or ester thereof or the solvate thereof according to any one of claims 1-5, **characterized in that** the R₁ has the following chemical structural formula: 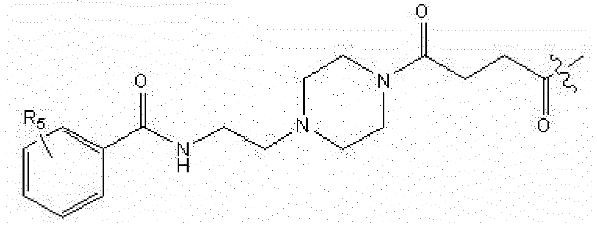
wherein R₅ is hydrogen, alkoxy containing 1 to 6 carbon atoms or alkyl containing 1 to 6 carbon atoms;
preferably, the alkoxy is alkoxy containing 1 to 3 carbon atoms; and the alkyl is alkyl containing 1 to 3 carbon atoms.

11. The ligand compound or the pharmaceutically acceptable salt or ester thereof or the solvate thereof according to any one of claims 1-5, **characterized in that** the R₁ has the following chemical structural formula: 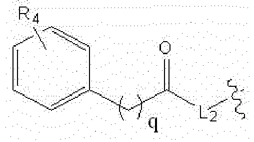
wherein R₄ is hydrogen, alkoxy containing 1 to 6 carbon atoms or alkyl containing 1 to 6 carbon atoms; q is any integer of 2 to 5; L₂ is an amino acid linker structure;
preferably, the alkoxy is alkoxy containing 1 to 3 carbon atoms; the alkyl is alkyl containing 1 to 3 carbon atoms; the amino acid in the amino acid linker structure is arginine, serine, histidine, lysine, glycine or glutamine; and the q is 2 or 3.

12. The ligand compound or the pharmaceutically acceptable salt or ester thereof or the solvate thereof according to any one of claims 1-5, **characterized in that** the R₁ has the following chemical structural formula: 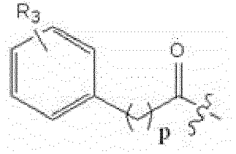
wherein R₃ is hydrogen, alkoxy containing 1 to 6 carbon atoms or alkyl containing 1 to 6 carbon atoms; p is any integer of 0 to 2;
preferably, the alkoxy is alkoxy containing 1 to 3 carbon atoms; the alkyl is alkyl containing 1 to 3 carbon atoms; and the p is 0 or 1.

13. The ligand compound or the pharmaceutically acceptable salt or ester thereof or the solvate thereof according to any one of claims 1-5, **characterized in that** the ligand compound is selected from the following compounds:

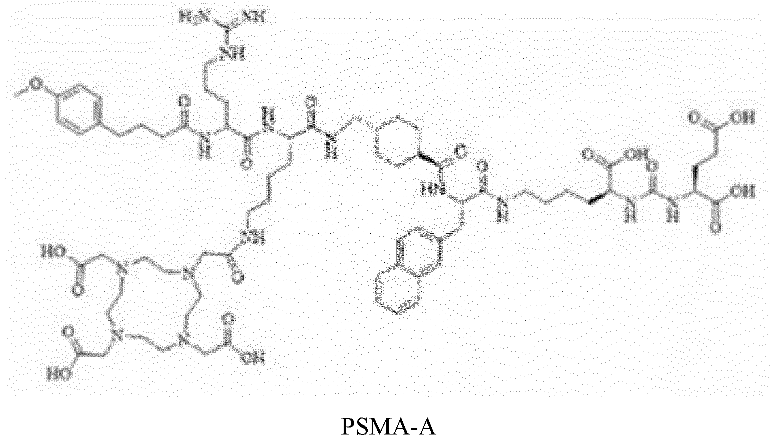

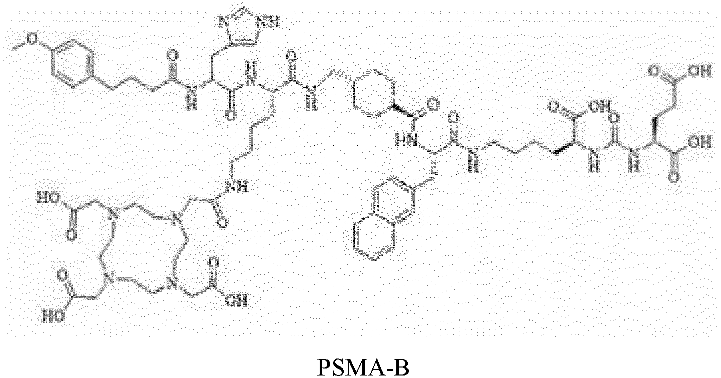

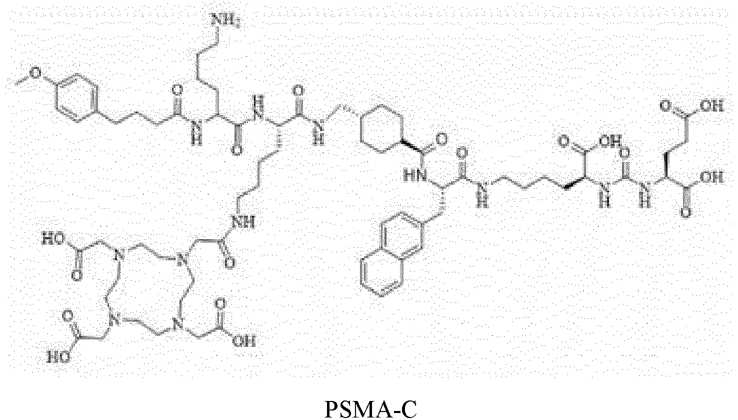

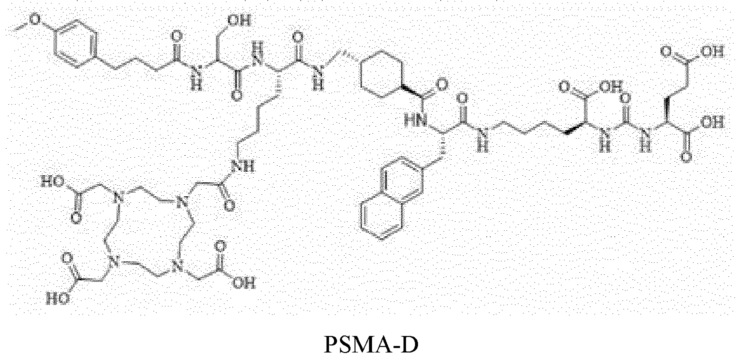

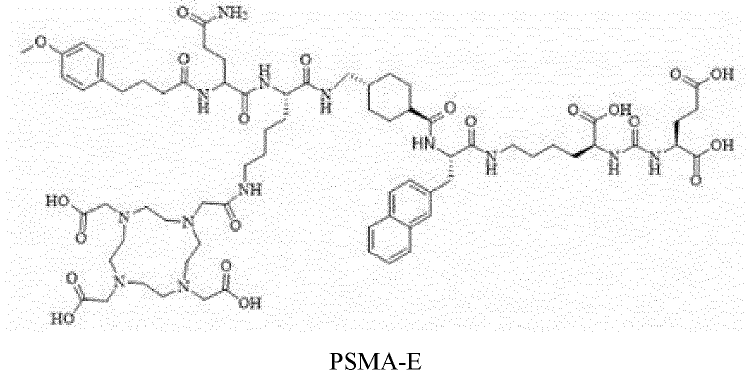

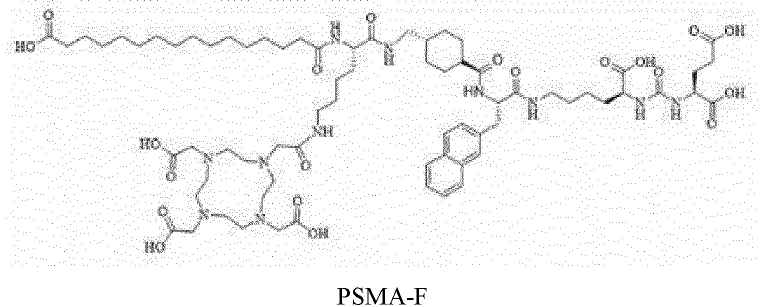

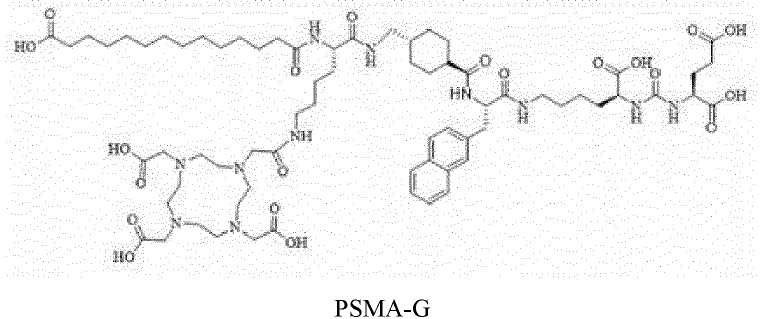

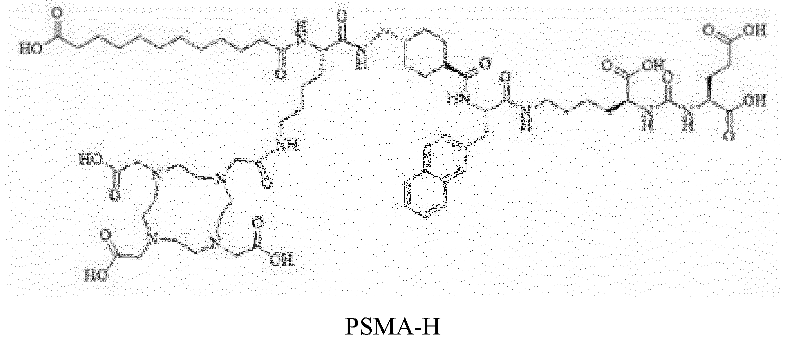

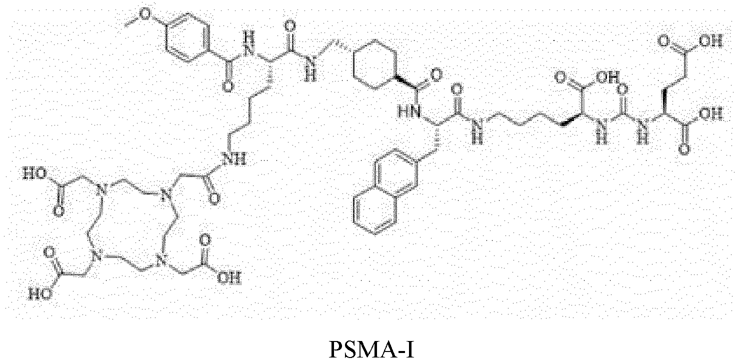

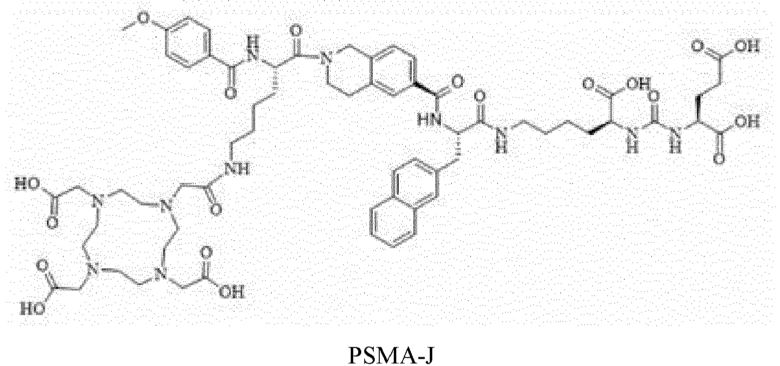

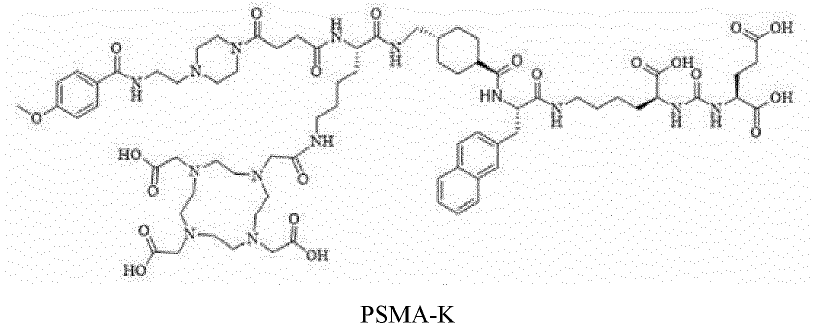

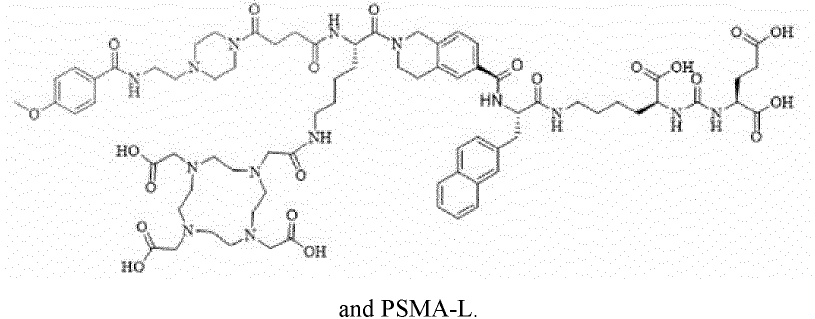

14. A ligand compound represented by the following structure or a pharmaceutically acceptable salt or ester thereof or a solvate thereof:

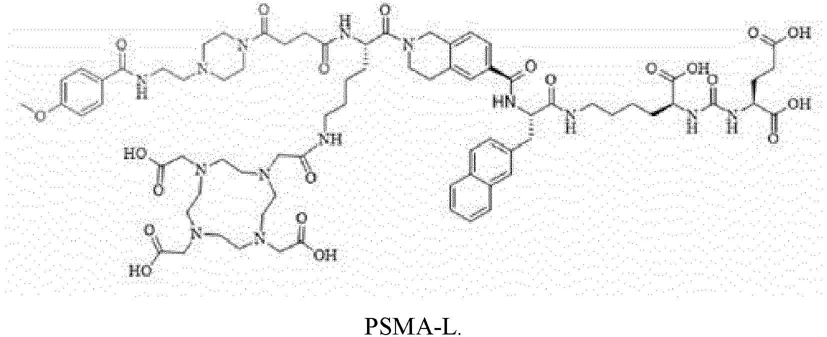

15. A chelate, **characterized in that** the chelate comprises the ligand compound or the pharmaceutically acceptable salt or ester thereof or the solvate thereof according to any one of claims 1-14, and a radioactive metal; the radioactive metal is complexed to the radioactive metal chelator group;
preferably, the radioactive metal is ⁶⁴Cu, ⁶⁷Cu, ⁹⁰Y, ¹¹¹In, ⁶⁸Ga, ^{117m}Sn, ¹⁵³Sm, ¹⁴⁹Tb, ¹⁶¹Tb, ¹⁷⁷Lu, ²²⁵Ac, ²¹³Bi, ²²⁴Ra, ²¹²Bi, ²¹²Pb, ²²⁵Ac, ²²⁷Th, ²²³Ra, ⁴⁷Sc, ¹⁸⁶Re, ¹⁸⁸Re, ⁶⁸Ga, ⁶⁴Cu, ¹¹¹In, ⁸⁹Zr, ⁴⁴Sc, ^{99m}Tc, ⁸⁶Y, ¹⁵²Tb or ¹⁵⁵Tb;
and more preferably, the radioactive metal is ¹⁷⁷Lu.

16. A chelate, **characterized in that** the chelate comprises ¹⁷⁷Lu and a ligand compound represented by the following structure or a pharmaceutically acceptable salt or ester thereof or a solvate thereof:

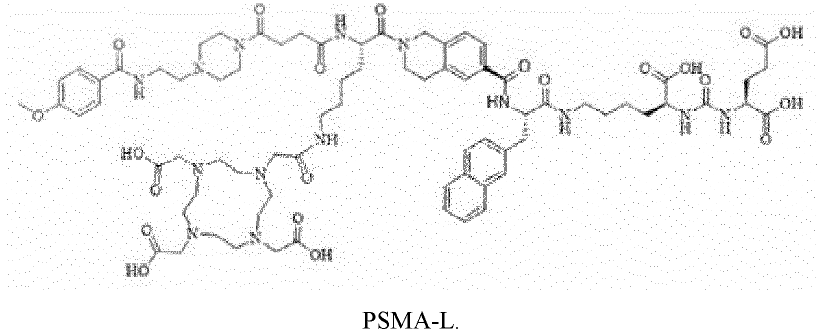

17. A composition, **characterized in that** the composition comprises the chelate according to claim 15 or 16; preferably, the composition further comprises a pharmaceutically acceptable excipient.

18. A cancer diagnosis or treatment reagent, **characterized in that** the cancer diagnosis or treatment reagent comprises the chelate according to claim 15 or 16; preferably, the cancer diagnosis or treatment reagent further comprises a pharmaceutically acceptable excipient.

19. The ligand compound or the pharmaceutically acceptable salt or ester thereof or the solvate thereof according to any one of claims 1-14, the chelate according to claim 15 or 16, or the composition according to claim 17 for use in the preparation of a cancer diagnosis or treatment reagent;
preferably, the cancer is a solid tumor or a hematological tumor;
more preferably, the cancer is prostate cancer;
and further preferably, the cancer is prostate-specific membrane antigen positive prostate cancer.

20. A method for imaging a patient with prostate cancer, **characterized in that** the method comprises the following steps: administering to the patient the chelate according to claim 15 or 16, the composition according to claim 17, the cancer diagnosis or treatment reagent according to claim 18, or a composition containing the cancer diagnosis or treatment reagent according to claim 18 and a pharmaceutically acceptable excipient, and performing tissue imaging;
preferably, the prostate cancer is prostate-specific membrane antigen positive prostate cancer;
and preferably, the imaging is PET imaging or SPECT imaging.

21. A method for treatment of a patient with prostate cancer, **characterized in that** the method comprises the following steps: administering to the patient the chelate according to claim 15 or 16, the composition according to claim 17, the cancer diagnosis or treatment reagent according to claim 18, or a composition containing the cancer diagnosis or treatment reagent according to claim 18 and a pharmaceutically acceptable excipient;
preferably, the prostate cancer is prostate-specific membrane antigen positive prostate cancer;
and preferably, the treatment is radiotherapy.
